(19) Europäisches Patentamt
European Patent Office
Office européen des brevets

(11) EP 0 579 424 B1

(12) # EUROPEAN PATENT SPECIFICATION

(45) Date of publication and mention of the grant of the patent:
**23.10.1996 Bulletin 1996/43**

(51) Int Cl.[6]: **C07D 239/36**, C07D 401/04, C07D 407/10, C07D 407/04, C07D 409/04, A01N 43/54

(21) Application number: **93305207.8**

(22) Date of filing: **02.07.1993**

(54) **2-substituted pyrimidines and herbicidal use thereof**

2-substituierte Pyrimidine und ihre Verwendung als Herbizide

Pyrimidines 2-substituées et leur utilisation comme herbicides

(84) Designated Contracting States:
**AT BE CH DE DK ES FR GB GR IE IT LI LU NL PT SE**

(30) Priority: **17.07.1992 US 916247**
**17.07.1992 US 916780**
**20.05.1993 US 62802**

(43) Date of publication of application:
**19.01.1994 Bulletin 1994/03**

(60) Divisional application: **95117397.0**

(73) Proprietor: **ROHM AND HAAS COMPANY**
**Philadelphia Pennsylvania 19105 (US)**

(72) Inventor: **Tice, Colin Michael**
**Melrose Park, Pennsylvania 19126 (US)**

(74) Representative: **Angell, David Whilton et al**
**ROHM AND HAAS (UK) LTD.**
**European Operations Patent Department**
**Lennig House**
**2 Mason's Avenue**
**Croydon CR9 3NB (GB)**

(56) References cited:
**EP-A- 058 822**
**DE-A- 2 249 163**
**GB-A- 2 130 214**
**US-A- 3 823 135**
**US-A- 3 833 582**

- **CHEMICAL ABSTRACTS, vol. 75, no. 8, 23 August 1971, Columbus, Ohio, US; abstract no. 55916g, P. LARDENOIS ET AL.**
- **CHEMICAL ABSTRACTS, vol. 79, no. 3, 23 July 1973, Columbus, Ohio, US; abstract no. 17909g, M.-T. MUSSETTA ET AL.**
- **CHEMICAL ABSTRACTS, vol. 100, no. 5, 30 January 1984, abstract no. 34489q**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 579 424 B1

## Description

This invention relates to novel 2-substituted pyrimidines and their use as broad spectrum herbicides.

Bull. Soc. Chim. Fr., 1971(5), pages 1858-68 and EP-A-0 058 822 disclose certain 2-arylpyrimidines. US-A-3,823,135 discloses certain herbicidal 2-halo-3-arylpyrimidines.

A need continues for novel and improved herbicidal compounds and compositions. This is particularly so since the targets of herbicides can become resistant to known herbicides over time and after use of such compositions. Additionally, economic and environmental considerations can favour herbicides having different modes of performance than those currently used.

Accordingly, one aspect the present invention provides compounds of the formula:

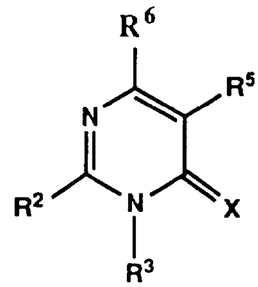

(I)

wherein

(a) $R^2$ is a furyl, phenyl, naphthyl, pyridyl, or thienyl group,

each of said groups is optionally substituted with up to three substituents independently selected from a bromo, chloro, fluoro, $(C_1\text{-}C_{12})$alkyl, cydo$(C_3\text{-}C_8)$alkyl, $(C_2\text{-}C_{12})$alkenyl, cyclo$(C_3\text{-}C_8)$alkenyl, $(C_2\text{-}C_{12})$alkynyl, halo $(C_1\text{-}C_{12})$alkyl, polyhalo$(C_1\text{-}_{C1}2)$alkyl, halo$(C_2\text{-}C_{12})$alkenyl, polyhalo$(C_2\text{-}C_{12})$alkenyl, halo$(C_2\text{-}C_6)$alkynyl, polyhalo (C2-C6)alkynyl, $(C_1\text{-}C_{12})$alkoxy, $(C_1\text{-}C_{12})$alkylthio, $(C_1\text{-}C_{12})$alkylsukfonyl, $(C_1\text{-}C_{12})$alkylsulfinyl, phenyl, phenyl$(C_1\text{-}C_{12})$alkyl, phenyl$(C_2\text{-}C_{12})$alkenyl, phenyl$(C_2\text{-}C_{12})$alkynyl, cyano, halo$(C_1\text{-}C_{12})$alkoxy, $(C_1\text{-}C_6)$alkoxy carbonyl, 1,3-dioxolan-2-yl, hydroxyimino, or nitro group; and when R2 is pyridyl, such pyridyl group is optionally substituted with oxygen on the nitrogen of the pyridyl group; or $R^2$ is a furyl, phenyl, naphthyl, pyridyl or thienyl group having a fused ring moiety composed of an oxymethyleneoxy or an oxoethyleneoxy link bonded to adjacent carbon atoms of said group;

(b) $R^3$ is a $(C_3\text{-}C_4)$alkenyl, $(C_5\text{-}C_6)$alkenynyl, $(C_3\text{-}C_6)$alkynyl, $(C_1\text{-}C_6)$alkoxy$(C_1\text{-}C_6)$alkyl, di$(C_1\text{-}C_6)$alkoxy$(C_1\text{-}C_6)$ alkyl, halo$(C_1\text{-}C_6)$alkoxy$(C_1\text{-}C_6)$alkyl, 2-oxo$(C_2\text{-}C_3)$alkyl, trimethylsilyl$(C_3\text{-}C_4)$alkynyl or cyano$(C_1\text{-}C_6)$alkyl group, each of said $(C_3\text{-}C_4)$alkenyl, or $(C_3\text{-}C_6)$alkynyl group being optionally substituted with up to five halogens; and

(c) $R^5$ is a hydrogen, $(C_1\text{-}C_5)$alkyl, $(C_3\text{-}C_6)$alkenyl, $(C_2\text{-}C_6)$alkynyl, halo$(C_1\text{-}C_6)$alkyl, polyhalo$(C_1\text{-}C_6)$alkyl, halo$(C_2\text{-}C_6)$alkenyl, polyhalo$(C_2\text{-}C_6)$alkenyl, halo$(C_2\text{-}C_6)$alkynyl, polyhalo$(C_2\text{-}C_6)$alkynyl, halo$(C_1\text{-}C_6)$alkoxy, polyhalo$(C_1\text{-}C_6)$alkoxy, trimethylsilyl$(C_2\text{-}C_3)$alkynyl, $(C_1\text{-}C_6)$alkoxy, $(C_1\text{-}C_6)$alkylthio, $(C_1\text{-}C_3)$alkoxycarbonyl$(C_1\text{-}C_3)$alkyl, halo, or cyano group; and

(d) $R^6$ is a hydrogen, halo, $(C_1\text{-}C_8)$alkyl, $(C_2\text{-}C_6)$alkenyl, $(C_2\text{-}C_6)$alkynyl, halo$(C_1\text{-}C_6)$alkyl, polyhalo$(C_1\text{-}C_6)$alkyl, halo$(C_2\text{-}C_6)$alkenyl, polyhalo$(C_2\text{-}C_6)$alkenyl, halo$(C_2\text{-}C_6)$alkynyl, polyhalo$(C_2\text{-}C_6)$alkynyl, $(C_1\text{-}C_6)$alkoxy, $(C_1\text{-}C_6)$ alkoxy$(C_1\text{-}C_4)$alkyl, $(C_1\text{-}C_6)$alkylthio, $(C_1\text{-}C_3)$alkoxycarbonyl, $(C_1\text{-}C_3)$alkoxycarbonyl$(C_1\text{-}C_3)$alkyl, $(C_6\text{-}C_{10})$aryl, $(C_6\text{-}C_1)$aryl $(C_1\text{-}C_4)$alkyl, cyclo$(C_3\text{-}C_7)$alkyl, $(C_4\text{-}C_5)$heterocyclyl selected from a group consisting of furyl, pyridyl and thienyl, $(C_1\text{-}C_3)$alkylamino, di$(C_1\text{-}C_3)$alkylamino, di$(C_1\text{-}C_3)$alkylaminocarbonyl, halo$(C_1\text{-}C_6)$alkylthio, polyhalo $(C_1\text{-}C_6)$alkythio, halo$(C_1\text{-}C_6)$alkoxy, polyhalo$(C_1\text{-}C_6)$alkoxy, $(C_6\text{-}C_{10})$aryloxy or cyano group; the aryl portion of said $(C_6\text{-}C_{10})$aryl, $(C_6\text{-}C_{10})$ aryl $(C_1\text{-}C_4)$alkyl and $(C_6\text{-}C_{10})$aryloxy groups being optionally substituted with up to three substituents independently selected from bromo, chloro, fluoro, $(C_1\text{-}C_{12})$alkyl, cyclo$(C_3\text{-}C_8)$alkyl, $(C_2\text{-}C_{12})$alkenyl, cyclo$(C_3\text{-}C_8)$alkenyl, $(C_2\text{-}C_{12})$alkynyl, halo$(C_1\text{-}C_{12})$alkyl, polyhalo$(C_1\text{-}C_{12})$alkyl, halo$(C_2\text{-}C_{12})$alkenyl, polyhalo$(C_2\text{-}C_{12})$alkenyl, halo$(C_2\text{-}C_6)$alkynyl, polyhalo$(C_2\text{-}C_6)$alkynyl, $(C_1\text{-}C_{12})$alkoxy, $(C_1\text{-}C_{12})$alkylthio, $(C_1\text{-}C_{12})$alkylsulfonyl, $(C_1\text{-}C_{12})$alkylsulfinyl, phenyl, phenyl$(C_1\text{-}C_{12})$alkyl, phenyl$(C_2\text{-}C_{12})$alkenyl, phenyl$(C_2\text{-}C_{12})$alkynyl, cyano, halo$(C_1\text{-}C_{12})$alkoxy, 1,3-dioxalan-2-yl, hydroxyimino, and nitro; and

(e) X is oxygen or sulfur.

It is to be understood that the prefix term "halo" designates a halogen substituent (such as fluorine, chlorine, bromine, or iodine) and that "polyhalo" designates two or more substituents independently selected from halogens. It

is further to be understood that, unless otherwise specified, use of the prefix "halo" without a concurrent use of the prefix "polyhalo" is not intended to limit the invention to singularly halogenated compounds. This invention also teaches methods of preparing these compounds as well as methods of using the compounds as herbicides.

In the compounds of the present invention,

$R^2$ is a furyl, phenyl, naphthyl, pyridyl, or thienyl, and each of said groups may be optionally substituted with up to three substituents independently selected from bromo: chloro; fluoro; $(C_1-C_{12})$alkyl, preferably$(C_1-C_6)$alkyl; cyclo$(C_3-C_8)$alkyl, preferably cyclo$(C_5-C_6)$alkyl; $(C_2-C_{12})$alkenyl, preferably $(C_2-C_6)$alkenyl; cyclo$(C_3-C_8)$alkenyl; $(C_2-C_{12})$alkynyl, preferably $(C_2-C_6)$alkynyl, halo$(C_1-C_{12})$alkyl, preferably halo$(C_1-C_6)$alkyl; polyhalo$(C_1-C_{12})$alkyl, preferably polyhalo$(C_1-C_6)$alkyl; halo$(C_2-C_{12})$alkenyl, preferably halo$(C_2-C_6)$alkenyl; polyhalo$(C_2-C_{12})$alkenyl, preferably polyhalo $(C_2-C_6)$alkenyl; halo$(C_2-C_6)$alkynyl; polyhalo$(C_2-C_6)$alkynyl; $(C_1-C_{12})$alkoxy, preferably $(C_1-C_6)$alkoxy; $(C_1-C_{12})$alkylthio, preferably $(C_1-C_6)$alkylthio; $(C_1-C_{12})$alkylsulfonyl; $(C_1-C_{12})$alkylsulfinyl; phenyl; phenyl$(C_1-C_{12})$alkyl; phenyl$(C_2-C_{12})$ alkenyl; phenyl$(C_2-C_{12})$alkynyl; cyano; halo$(C_1-C_{12})$alkoxy, preferably halo$(C_1-C_6)$alkoxy; $(C_1-C_6)$alkoxyl carbonyl, 3-dioxalan-2-yl; hydroxyimino, and nitro. Substituent groups can be branched or unbranched. Preferred phenyl groups are phenyl, 3-methylphenyl, 3-methoxyphenyl, 3-nitrophenyl, 4-fluorophenyl, 4-chlorophenyl, 3-trifluoromethylphenyl, 3-bromophenyl, 3-chlorophenyl, 3-fluorophenyl, 3-trifluoromethoxyphenyl, 3-cyanophenyl, 3-(1,3-dioxolan-2-yl)phenyl, 3-(hydroxyimino)phenyl, 2-fluorophenyl, 2-chlorophenyl, 2-trifluoromethoxyphenyl, 3,5-difluorophenyl, 3,5-dichlorophenyl, 2,4-difluorophenyl, 2,5-difluorophenyl, 3-chloro-4-fluorophenyl, 3,4-difluorophenyl, 3-fluoro-5-trifluoromethylphenyl, 3,4,5-trifluorophenyl; more preferably phenyl, 3-fluorophenyl, and 3-chlorophenyl. Preferred pyridyl groups are 6-chloro-2-pyridyl, 3-pyridyl, 1-methyl-3-pyridinium, 5-bromo-3-pyridyl, 5,6-dichloro-3-pyridyl, 5-chloro-3-pyridyl, 1-oxo-3-pyridyl, 4-pyridyl, 2-fluoro-4-pyridyl, 2-chloro-4-pyridyl, 2-chloro-6-methyl-4-pyridyl, 2-methyl-4-pyridyl, 2-methoxy-4-pyridyl, 2-cyano-4-pyridyl,1 -oxo-4-pyridyl, 2,6-difluoro-4-pyridyl and 2,6-dichloro-4-pyridyl. More preferred are 2-chloro-4-pyridyl, 2-fluoro-4-pyridyl, and 2,6-dichloro-4-pyridyl. The pyridyl groups can also be present as a salt, such as 1-methyl-3-pyridinium iodide or 3-pyridinium hydrochloride. Preferred furyl groups are 2-furyl and 3-furyl. A preferred naphthyl group is 2-naphthyl. Preferred thienyl groups are 2-thienyl, 3-thienyl, 4-chloro-2-thienyl, 5-chloro-2-thienyl, 5-chloro-3-thienyl and 2,5-dichloro-3-thienyl. In one embodiment of the present invention $R^2$ is 2-furyl, phenyl, 3-fluorophenyl, 3-chlorophenyl, 3,5-difluorophenyl, 3,5-dichlorophenyl, 2-chloro-4-pyridyl, 2-fluoro-4-pyridyl or 2,6-dichloro-4-pyridyl.

In the case of $R^2$ being a pyridyl group, an additional selection of substituent groups is oxygen substituted on the nitrogen atom of the pyridyl ring; e.g. N-oxo groups, such as 1-oxo-3-pyridyl or 1-oxo-4-pyridyl. Optionally, each of the furyl, phenyl, naphthyl, pyridyl and thienyl groups can have a fused ring moiety such that the fused ring is composed of an oxymethyleneoxy ($-O-CH_2-O-$) link or an oxyethyleneoxy ($-O-CH_2CH_2-O-$) link which is bonded to adjacent carbon atoms of the group. For example, 3,4-methylenedioxyphenyl.

$R^3$ is: a $(C_3-C_4)$alkenyl or $(C_3-C_6)$alkynyl group, each of which may be optionally substituted with up to five halogens; or $R^3$ is a $(C_5-C_6)$ alkenynyl; $(C_1-C_6)$alkoxy$(C_1-C_6)$alkyl, di$(C_1-C_6)$alkoxy$(C_1-C_6)$alkyl, halo$(C_1-C_6)$alkoxy$(C_1-C_6)$alkyl, 2-oxo$(C_2-C_3)$alkyl, trimethylsilyl$(C_3-C_4)$alkynyl or cyano$(C_1-C_6)$alkyl group. A preferred $(C_1-C_3)$alkyl group is ethyl. Preferred alkenyl and halogen substituted alkenyl groups are allyl and 3-chloroallyl. Preferred alkynyl groups are pentynyl, propynyl and butynyl, more preferably pent-2-ynyl, prop-2-ynyl, and but-2-ynyl. Preferred halogen substituted $(C_3-C_6)$ alkynyl groups are iodopropargyl and bromopropargyl. Preferred $(C_1-C_6)$alkoxy$(C_1-C_6)$alkyls are $(C_1-C_2)$alkoxy$(C_1-C_3)$ alkyl, more preferably methoxymethyl and 2-methoxyethyl, and most preferably methoxymethyl. Preferred di$(C_1-C_6)$ alkoxy$(C_1-C_6)$alkyls are di$(C_1-C_2)$alkoxy$(C_1-C_3)$alkyls, more preferably 2,2-dimethoxypropyl. A preferred 2-oxo$(C_2-C_3)$ alkyl is acetonyl. A preferred trimethylsilyl $(C_3-C_4)$alkynyl is 3-(trimethylsilyl)propargyl. A preferred cyano $(C_1-C_6)$alkyl is cyanomethyl. A preferred $(C_5-C_6)$alkenynyl, is pent-4-en-2-ynyl. In one embodiment of the present invention $R^3$ is allyl, pent-2-ynyl, prop-2-ynyl, but-2-ynyl, methoxymethyl, 2-methoxyethyl, acetonyl, 2,2-dimethoxypropyl, pent-4-en-2-ynyl, 3-chloroallyl, 3-iodopropargyl, 3-trimethylsilyl, propargyl or cyanomethyl.

$R^5$ is hydrogen, $(C_1-C_5)$alkyl, $(C_3-C_6)$alkenyl, $(C_2-C_6)$alkynyl, trimethylsilyl$(C_2-C_3)$alkynyl, $(C_1-C_6)$alkoxy, halo- or polyhalo$(C_1-C_6)$alkyl, halo- or polyhalo$(C_2-C_6)$alkenyl, halo- or polyhalo$(C_2-C_6)$alkynyl, halo $(C_1-C_6)$alkoxy, polyhalo $(C_1-C_6)$alkoxy, $(C_1-C_3)$alkoxycarbonyl$(C_1-C_3)$alkyl, $(C_1-C_6)$alkylthio, halo or cyano. Preferred $(C_1-C_5)$alkyls are methyl, ethyl, n-propyl and iso-propyl, more preferably methyl and ethyl. Preferred $(C_2-C_6)$alkynyls are $(C_2-C_4)$alkynyls, more preferably prop-2-ynyl. Preferred $(C_1-C_6)$alkoxys are $(C_1-C_2)$alkoxys, more preferably methoxy. Preferred $(C_1-C_6)$ alkylthios are $(C_1-C_2)$alkylthiol, more preferably methylthio. A preferred $(C_1-C_3)$alkoxycarbony$(C_1-C_3)$alkyl is methoxycarbonylmethyl. Preferred $(C_3-C_6)$alkenyls are $(C_3-C_4)$alkenyl, more preferably allyl. Preferred halo$(C_1-C_6)$alkyls and polyhalo$(C_1-C_6)$alkyls are halo$(C_1-C_2)$alkyls and polyhalo$(C_1-C_2)$alkyls, more preferably fluoromethyl and trifluoromethyl. Preferred halo$(C_1-C_6)$alkoxys and polyhalo$(C_1-C_6)$alkoxys are halo$(C_1-C_2)$alkoxys, and polyhalo$(C_1-C_2)$alkoxys, more preferably difluoromethoxy and trifluoromethoxy. Preferred halos are chloro and fluoro. A preferred trimethylsilyl $(C_2-C_3)$alkynyl is trimethylsilylethynyl. In one embodiment of the present invention $R^5$ is hydrogen, fluoro, chloro, methyl, ethyl, n-propyl, i-propyl, prop-2-ynyl, trimethysilylethynyl, methylthio, methoxy, methoxycarbonylmethyl, allyl, fluoromethyl, or trifluoromethyl.

$R^6$ is hydrogen, halo, straight $(C_1-C_8)$alkyl, branched $(C_3-C_8)$alkyl, $(C_2-C_6)$alkenyl, $(C_2-C_6)$alkynyl, halo$(C_1-C_6)$alkyl

or polyhalo($C_{1-6}$)alkyl, halo($C_2$-$C_6$)alkenyl or polyhalo($C_2$-$C_6$)alkenyl, halo($C_2$-$C_6$)alkynyl or polyhalo($C_2$-$C_6$)alkynyl, ($C_1$-$C_6$)alkoxy($C_1$-$C_4$)alkyl, ($C_1$-$C_6$)alkoxy, ($C_1$-$C_6$)alkythio, ($C_1$-$C_3$)alkoxycarbonyl, ($C_1$-$C_3$)alkoxycarbonyl($C_1$-$C_3$) alkyl, substituted or unsubstituted ($C_6$-$C_{10}$)aryl, substituted or unsubstituted ($C_6$-$C_{10}$)alkoxys, substituted or unsubstituted ($C_6$-$C_{10}$) aryl ($C_1$-$C_4$)alkyl, cyclo($C_3$-$C_7$)alkyl, halo($C_1$-$C_6$)alkylthio, polyhalo ($C_1$-$C_6$)alkythio, halo($C_1$-$C_6$)alkoxy, polyhalo ($C_1$-$C_6$)alkoxy, ($C_4$-$C_5$)heterocyclyl, ($C_1$-$C_3$)alkylamino, di($C_1$-$C_3$)alkylamino, di($C_1$-$C_3$)alkyl aminocarbonyl, and cyano. In $R^6$ the aryl portion of the ($C_6$-$C_{10}$)aryl, ($C_6$-$C_{10}$)aryloxy and ($C_6$-$C_{10}$)aryl($C_1$-$C_4$)alkyl groups can be optionally substituted with up to three substituents independently selected from bromo; chloro; fluoro; ($C_1$-$C_{12}$)alkyl, preferably ($C_1$-$C_6$)alkyl; cyclo($C_3$-$C_8$)alkyl, preferably cyclo($C_5$-$C_6$)alkyl; ($C_2$-$C_{12}$)alkenyl, preferably ($C_2$-$C_6$)alkenyl; cyclo($C_3$-$C_8$)alkenyl; ($C_2$-$C_{12}$)alkynyl, preferably ($C_2$-$C_6$)alkynyl; halo($C_1$-$C_{12}$)alkyl, preferably halo($C_1$-$C_6$)alkyl; polyhalo ($C_1$-$C_{12}$)alkyl, preferably polyhalo($C_1$-$C_6$)alkyl; halo($C_2$-$C_{12}$)alkenyl, preferably halo($C_2$-$C_6$)alkenyl; polyhalo($C_2$-$C_{12}$) alkenyl, preferably polyhalo($C_2$-$C_6$)alkenyl; halo($C_2$-$C_6$)alkynyl; polyhalo($C_2$-$C_6$)alkynyl; ($C_1$-$C_{12}$)alkoxy, preferably ($C_1$-$C_6$)alkoxy; ($C_1$-$C_{12}$)alkylthio, preferably ($C_1$-$C_6$)alkylthio; ($C_1$-$C_{12}$)alkylsulfonyl; ($C_1$-$C_{12}$)alkylsulfinyl; phenyl; phenyl($C_1$-$C_{12}$)alkyl; phenyl($C_2$-$C_{12}$)aSkenal; phenyl($C_2$-$C_{12}$)alkynyl; cyano; halo($C_1$-$C_{12}$)alkoxy, preferably halo($C_1$-$C_6$) alkoxy; 1,3-dioxalan-2-yl; hydroxyimino; and nitro. Preferred ($C_1$-$C_8$)alkyls are straight ($C_1$-$C_7$)alkyls and branched ($C_3$-$C_8$)alkyls, preferably methyl, ethyl, n-propyl, n-butyl, n-pentyl, n-hexyl, n-heptyl, s-butyl, i-propyl, i-butyl and t-butyl; more preferably methyl, ethyl, n-propyl, s-butyl, i-propyl and t-butyl. A preferred ($C_2$-$C_6$)alkenyl is 2-methyl-1-propenyl. A preferred ($C_6$-$C_{10}$)aryl is phenyl. A preferred ($C_6$-$C_{10}$)aryloxy is phenoxy. Preferred ($C_4$-$C_5$)heterocyclyls are 3-thienyl, 3-furyl, 2-thienyl and 4-pyridyl; most preferably 3-thienyl. Preferred ($C_1$-$C_6$)alkoxys are ($C_1$-$C_5$)alkoxys, more preferably methoxy and ethoxy. A preferred ($C_1$-$C_3$)alkoxycarbonyl is ethoxycarbonyl. Preferred ($C_2$-$C_6$)alkynyls are but-2-ynyl, but-3-ynyl, and prop-2-ynyl. Preferred halos are fluoro, bromo, and chloro; more preferably chloro and bromo. Preferred halo($C_1$-$C_6$)alkyls and polyhalo($C_1$-$C_6$)alkyls are halo($C_1$-$C_3$)alkyls and polyhalo($C_1$-$C_3$)alkyls, more preferably trifluoromethyl, pentafluoroethyl, trichloromethyl, bromomethyl, chloromethyl, difluoromethyl, and chlorodifluoromethyl; most preferably trifluoromethyl. Preferred halo($C_1$-$C_6$)alkoxys and polyhalo($C_1$-$C_6$)alkoxys are halo($C_1$-$C_3$)alkoxys and polyhalo($C_1$-$C_3$)alkoxys, more preferably difluoromethoxy and trifluoromethoxy. Preferred ($C_1$-$C_6$)alkylthios are ($C_1$-$C_5$) alkylthiol, more preferably methylthio. A preferred ($C_1$-$C_6$)alkoxy($C_1$-$C_4$)alkyl is methoxymethyl. A preferred aryl ($C_1$-$C_4$)alkyl is benzyl. Preferred cyclo($C_3$-$C_7$)alkyls are cyclopropyl, cyclobutyl and cyclopentyl. A preferred di($C_1$-$C_3$) alkylamino is dimethylamino. A preferred di($C_1$-$C_3$)alkylaminocarbonyl is dimethylaminocarbonyl.

X is oxygen or sulfur, preferably oxygen.

A preferred embodiment of this invention are the compounds represented by formula I wherein X is oxygen and $R^2$ is substituted or unsubstituted phenyl, pyridyl, or thienyl.

A more preferred embodiment of this invention are the compounds represented by formula I wherein X is oxygen; $R^2$ is substituted or unsubstituted phenyl, substituted or unsubstituted pyridyl or substituted or unsubstituted thienyl; and $R^3$ is ($C_3$-$C_6$)alkynyl.

A still more preferred embodiment of this invention are the compounds represented by formula I wherein X is oxygen; $R^2$ is substituted or unsubstituted phenyl, substituted or unsubstituted pyridyl or substituted or unsubstituted thienyl; $R^3$ is ($C_3$-$C_6$)alkynyl; and $R^5$ and $R^6$ are independently selected from hydrogen, halo, ($C_1$-$C_4$)alkyl, polyhalo ($C_1$-$C_4$)alkyl, and ($C_1$-$C_4$)alkoxy. $R^6$ can be also unsubstituted or substituted phenyl.

Even more preferred are the compounds represented by formula I wherein X is oxygen; $R^2$ is phenyl, 3-substituted phenyl (i.e. meta-substituted phenyl), 3,5-disubstituted-phenyl or 3,4,5-trisubstituted phenyl, 2-substituted-4-pyridyl or or 3-thienyl or 5-substituted-3-thienyl; $R^3$ is ($C_3$-$C_6$)alkynyl; and $R^5$ and $R^6$ are independently selected from hydrogen, halo, ($C_1$-$C_4$)alkyl, polyhalo($C_1$-$C_4$)alkyl and ($C_1$-$C_4$)alkoxy. $R^6$ can be also unsubstituted or substituted phenyl.

A yet more preferred embodiment of this invention are the compounds represented by formula I wherein X is oxygen; $R^2$ is phenyl, 3-fluorophenyl, 3-chlorophenyl, 3,5-difluorophenyl, 3,5-dichlorophenyl, 3,4,5-trifluorophenyl, 2-chloro-4-pyridyl, 2-fluoro-4-pyridyl, 2,6-dichloro-4-pyridyl or 3-thienyl or 5-chloro-3-thienyl; $R^3$ is propargyl; $R^5$ is hydrogen, methyl, ethyl, methoxy, fluoro or chloro; and $R^6$ is methyl, ethyl, isopropyl, n-propyl, n-butyl, s-butyl, i-butyl, t-butyl, trifluoromethyl, difluoromethyl, phenyl, chloro, bromo, or fluoro.

Preferred compounds include

(a) 5,6-diethyl-2-phenyl-3-propargyl-4(3H)-pyrimidinone;
(b) 5-ethyl-2-phenyl-3-propargyl-6-trifluoromethyl-4(3H)-pyrimidin one;
(c) 5-ethyl-6-(1-methylethyl)-2-phenyl-3-propargyl-4(3H)-pyrimidin one;
(d) 6-chloro-5-ethyl-2-phenyl-3-propargyl-4(3H)-pyrimidinone;
(e) 5,6-diethyl-2-(3-fluorophenyl)-3-propargyl-4(3H)-pyrimidinone;
(f) 2-(2,6-dichloro-4-pyridyl)-5,6-diethyl-3-propargyl-4(3H)-pyrimidi none;
(g) 5,6-diethyl-2-(3,5-difluorophenyl)-3-propargyl-4(3H)-pyrimidinone;
(h) 5-ethyl-2-phenyl-3-propargyl-6-(n-propyl)-4(3H)-pyrimidinone.
(i) 6-difluoromethyl-5-ethyl-2-phenyl-3-propargyl-4(3H)-pyrimidin one;
(j) 6-ethyl-5-methoxy-2-phenyl-3-propargyl-4(3H)- pyrimidinone;

In a further aspect the invention provides a herbicidal composition comprising a herbicidally effective amount of a compound as defined above, and an agronomically acceptable carrier. Another aspect of the invention is a method of controlling a weed comprising applying a herbicidally effective amount of a composition or compound as defined above to said weed or to the locus of said weed or to the growth medium of said weed. The invention also comprises in a still further aspect the use of a compound or composition as defined above as a herbicide.

Compounds encompassed by the present invention include, but are not limited to, those illustrated in Table 1. The synthesis methods (i.e., "A", "B" etc.) specified in the table are described hereinafter in this specification. The sequence of letters in the "Synthesis" column indicates the relative sequence of steps performed. For instance, "D + A" indicates the steps of procedure D were first performed, followed by the steps of Procedure A.

TABLE 1

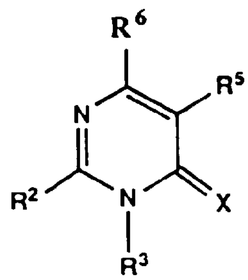

For the below table, "Me" is methyl, "Et" is ethyl, "Pr" is propyl, "Bu" is butyl, "Pe" is pentyl, "Bn" is benzyl, and "Ph" is phenyl; and except as noted, X = O (oxygen).

| Compound No. | $R^2$ | $R^3$ | $R^5$ | $R^6$ | Mpt °C | Synthesis |
|---|---|---|---|---|---|---|
| 1 | -Ph | $-CH_2C{\equiv}CH$ | -Me | -Me | 125-128 | D + A |
| 2 | -Ph | $-CH_2C{\equiv}CH$ | -H | -Me | 148-149 | D + A |
| 4 | -Ph | $-CH_2C{\equiv}CH$ | -H | $-CF_3$ | 118-120 | D + A |
| 5 | -Ph | $-CH_2C{\equiv}CH$ | -Me | -Et | 115-117 | D + A |
| 6 | -Ph | $-CH_2OCH_3$ | -H | $-CF_3$ | 89-90 | D + Z6 |
| 7 | -Ph | $-CH_2C{\equiv}CH$ | -Br | $-CF_3$ | 156-160 | D+Y1+A |
| 8 | -Ph | $-CH_2C{\equiv}CH$ | -Me | -Ph | 170-173 | D + A |
| 9 | -Ph | $-CH_2C{\equiv}CH$ | -Cl | -Me | 131-134 | D + A |
| 11 | -Ph | $-CH_2C{\equiv}CH$ | -H | $-t-C_4H_9$ | Oil | D + A |
| 12 | -Ph | $-CH_2C{\equiv}CH$ | -H | -Pr | 72.5-74 | D + A |
| 13 | -Ph | $-CH_2C{\equiv}CH$ | -i-Pr | -Me | 79-80.5 | D + A |
| 14 | -Ph | $-CH_2C{\equiv}CH$ | -Et | -Me | 103-106 | D + A |
| 16 | -Ph | $-CH_2C{\equiv}CH$ | -Me | -Cl | 137.5-139 | E + H + I + A |
| 17 | -Ph | $-CH_2C{\equiv}CH$ | -n-Pe | -Me | 92-93.5 | D + A |
| 18 | -Ph | $-CH_2CH{=}CH_2$ | -H | $-CF_3$ | Oil | D + A |
| 19 | -Ph | $-CH_2C{\equiv}CH$ | -Me | $-C(O)NMe_2$ | 137-140 | D + A +Z1 |
| 21 | -Ph | $-CH_2C{\equiv}CCH_2CH_3$ | -H | $-CF_3$ | 99-100.5 | D + A |
| 22 | -Ph | $-CH_2C{\equiv}CH$ | -Et | -Et | 101-103 | D + A |
| 23 | -Ph | $-CH_2C{\equiv}CH$ | -Me | $-NMe_2$ | 111.5-113.5 | E+H+I+A+Z2 |
| 25 | -Ph | $-CH_2C(O)Me$ | -Me | $-C_2H_5$ | 87-89 | D + Z3 |
| 26 | -Ph | $-CH_2C{\equiv}CH$ | -Me | -SMe | 156-159 | E+H+I+A+Z4 |
| 27 | -Ph | $-CH_2C{\equiv}CH$ | -H | $-C_2F_5$ | 134-136 | D + A |

| | | | | | | |
|---|---|---|---|---|---|---|
| 28 | -Ph | $-CH_2C{\equiv}CH$ | -Et | -Ph | 124-126 | D + A |
| 29 | -Ph | $-CH_2C{\equiv}CH$ | -Me | $-CF_3$ | 143-145 | D + A |
| 30 | -Ph | $-CH_2C{\equiv}CH$ | -n-Pr | $-CF_3$ | 155-157 | D + A |
| 31 | -Ph | $-CH_2C{\equiv}CH$ | -OMe | $-CF_3$ | 103-106 | D + A |
| 32 | -Ph | $-CH_2C{\equiv}CH$ | -H | $-C_2H_5$ | 101-103 | D + A |
| 33 | -Ph | $-CH_2C{\equiv}CH$ | -Me | $-CH_2OMe$ | 125-127 | D + A |
| 35 | -4-ClPh | $-CH_2C{\equiv}CH$ | -Me | -Et | 109-111 | D + A |
| 36 | -Ph | $-CH_2C(OMe)_2Me$ | -Me | -Et | 85-88 | D + A + Z5 |
| 37 | -Ph | $-CH_2CH_2OMe$ | -H | $-CF_3$ | 49-51 | B |
| 38 | -Ph | $-CH_2C{\equiv}CH$ | -CN | -SMe | 189-191 | A |
| 39 | -Ph | $-CH_2C{\equiv}CCH_3$ | -H | $-CF_3$ | 85-88 | D + A |
| 40 | -Ph | $-CH_2C{\equiv}CH$ | -F | -Cl | 113-115 | D + H + I + A |
| 45 | -Ph | $-CH_2C{\equiv}CH$ | -H | $-CCl_3$ | 86-91 | D + A |
| 46 | -Ph | $-CH_2C{\equiv}CH$ | -Et | $-CF_3$ | 113-115 | B or D + A |
| 47 | -Ph | $-CH_2C{\equiv}CH$ | -SMe | $-CF_3$ | 159-162 | D + A |
| 48 | -Ph | $-CH_2C{\equiv}CH$ | -Me | i-Pr | 120-122 | D + A |
| 51 | -Ph | $-CH_2C{\equiv}CH$ | -Me | -H | 135-136 | D + A |
| 52 | -Ph | $-CH_2C{\equiv}CH$ | -Me | $-CO_2Et$ | 147-150 | D+A |
| 53 | -Ph | $-CH_2CH{=}CH_2$ | -Et | $-CF_3$ | 73-76 | B |
| 54 | -2-Cl-4-pyridyl | $-CH_2C{\equiv}CH$ | -Et | $-CF_3$ | 82-84 | B |
| 55 | -Ph | $-CH_2C{\equiv}CH$ | -Et | -n-Bu | 57-59 | D+ A |
| 56 | -2-Cl-4-pyridyl | $-CH_2C{\equiv}CH$ | -Et | -Et | 84-86 | D+A |
| 57 | -3,5-diClPh | $-CH_2C{\equiv}CH$ | -Et | -Et | 113-114 | D+ A |
| 58 | -4-pyridyl | $-CH_2C{\equiv}CH$ | -Et | $-CF_3$ | 114-116 | B |
| 59 | -2-Cl-6-Me-4-pyridyl | $-CH_2C{\equiv}CH$ | -Et | -Et | 119-121 | D+A |
| 60 | -3-pyridyl | $-CH_2C{\equiv}CH$ | -Et | $-CF_3$ | 116-118 | B |

| | | | | | | |
|---|---|---|---|---|---|---|
| 61 | -1-Me-3-pyridinium iodide | | | | | |
| | | $-CH_2C≡CH$ | -Et | $-CF_3$ | >170(dec) | B+Z13 |
| 62 | -2-F-4-pyridyl | $-CH_2C≡CH$ | -Et | $-CF_3$ | Oil | B |
| 63 | -5,6-diCl-3-pyridyl | | | | | |
| | | $-CH_2C≡CH$ | -Et | $-CF_3$ | 89-92 | B |
| 64 | -3,4-diFPh | $-CH_2C≡CH$ | -Et | -Et | 97-99 | D+A |
| 65 | -2,6-diCl-4-pyridyl | | | | | |
| | | $-CH_2C≡CH$ | -Et | $-CF_3$ | 129-131 | B |
| 66 | -5-Br-3-pyridyl | $-CH_2C≡CH$ | -Et | $-CF_3$ | 123-125 | B |
| 67 | -2,6-diCl-4-pyridyl | | | | | |
| | | $-CH_2C≡CH$ | -Et | i-Pr | solid | D+A |
| 68 | -4-FPh | $-CH_2C≡CH$ | -Et | -Et | 107-109 | D+A |
| 69 | -3-formylPh | $-CH_2C≡CH$ | -Et | -Et | 82-86 | D+A+Z14 |
| 70 | -2,4-diFPh | $-CH_2C≡CH$ | -Et | -Et | 68-71 | D+A |
| 71 | -2,5-diFPh | $-CH_2C≡CH$ | -Et | -Et | 99-102 | D+ A |
| 76 | -Ph | $-CH_2C≡CH$ | -Cl | $-CF_3$ | 135-138 | D+ A |
| 77 | -3-ClPh | $-CH_2C≡CH$ | -H | $-CF_3$ | 103-106 | D+ A |
| 80 | -2-thienyl | $-CH_2C≡CH$ | -H | -Me | 103.5-105 | A |
| 81* | -Ph | $-CH_2C≡CH$ | -H | $-CH_2Cl/-CH_2Br$ mixture | | |
| | | | | | solid | A |
| 83 | -3-thienyl | $-CH_2C≡CH$ | -H | -Et | 97-99 | A |
| 84 | -Ph | $-CH_2C≡CH$ | $-CH_2C≡CH$ | -Et | 128-130 | D+A |
| 86 | -Ph | $-CH_2C≡CH$ | -Et | -i-Pr | 92-94 | D+A |
| 87 | $-3-NO_2-Ph$ | $-CH_2C≡CH$ | -Et | -Et | 114-119 | D+ A |
| 88 | $-3-CF_3-Ph$ | $-CH_2C≡CH$ | -Et | -Et | 67-70 | D+A |
| 89 | -Ph | $-CH_2C≡CH$ | -Br | -Ph | 166-169 | D+Y1+ A |
| 90 | -3-FPh | $-CH_2C≡CH$ | -Et | -Et | 114-116 | D+A |

| | | | | | | |
|---|---|---|---|---|---|---|
| 91 | -Ph | $-CH_2C{\equiv}CH$ | -I | -Et | 175-177 | D+Y2+ A |
| 92 | -Ph | $-CH_2C{\equiv}CH$ | $-C{\equiv}CSiMe_3$ | -Et | Oil | D+Y2+ A+Z7 |
| 94 | -2-FPh | $-CH_2C{\equiv}CH$ | -Et | -Et | 76.5-79 | D+A |
| 95 | -3-MePh | $-CH_2C{\equiv}CH$ | -Et | -Et | 70-73 | D+A |
| 96 | -3-MeO-Ph | $-CH_2C{\equiv}CH$ | -Et | -Et | 72-75 | D+A |
| 97 | -Ph | $-CH_2C{\equiv}CH$ | -Et | -Br | 94-95 | E+H+I+ A |
| 98 | -3-ClPh | $-CH_2C{\equiv}CH$ | -Et | -Et | 109-111.5 | D+ A |
| 99 | -3-thienyl | $-CH_2C{\equiv}CH$ | -Et | -Et | 123-126.5 | D+ A |
| 103 | -Ph | $-CH_2C{\equiv}CH$ | -Et | -n-Pr | 82.5-85 | D+ A |
| 105 | -Ph | $-CH_2C{\equiv}CH$ | $-CH_2CO_2Me$ | -Et | 153-154 | D+A |
| 106 | -Ph | $-CH_2C{\equiv}CH$ | -Et | -Cl | 108-109.5 | E+H+I+A |
| 107 | -Ph | $-CH_2C{\equiv}CH$ | -Et | -F | 132-134 | E+H+Y3+I+A |
| 108 | -Ph | $-CH_2C{\equiv}CH$ | -F | -Et | 95-99.5 | D+A |
| 110 | -2,6-diCl-4-pyridyl | $-CH_2C{\equiv}CH$ | -Et | -Et | 140-142 | D+ A |
| 111 | -Ph | $-CH_2C{\equiv}CCH{=}CH_2$ | -Et | $-CF_3$ | 104-106 | B+Z12 |
| 112 | -Ph | $-CH_2C{\equiv}CH$ | -Et | -3-furyl | 114-115 | D+A |
| 114 | -Ph | $-CH_2C{\equiv}CH$ | -Et | -2-thienyl | 133-135 | D+A |
| 116 | -3-FPh | $-CH_2C{\equiv}CH$ | -Et | $-CF_3$ | 102- 105 | |
| 117 | -Ph | $-CH_2C{\equiv}CH$ | -Et | -3-thienyl | 82-85 | D+A |
| 118 | -3-FPh | $-CH_2C{\equiv}CH$ | -Et | -i-Pr | 124.5-127.5 | D+ A |
| 119 | -3-ClPh | $-CH_2C{\equiv}CH$ | -Et | -i-Pr | 112-115 | D+ A |
| 120 | -Ph | $-CH_2C{\equiv}CH$ | -Et | -4-pyridyl | Oil | D+ A |
| 121 | -Ph | $-CH_2C{\equiv}CH$ | -Et | -cyclo-Bu | 113-115 | D+A |
| 122 | -Ph | $-CH_2C{\equiv}CH$ | -Et | $-CH_2Ph$ | 105-107 | D+ A |
| 123 | -3,5-diFPh | $-CH_2C{\equiv}CH$ | -Et | -Et | 125-126.5 | D+ A |

| | | | | | | |
|---|---|---|---|---|---|---|
| 125 | -Ph | $-CH_2C\equiv CH$ | -Et | $-CF_2Cl$ | 115-117 | D+A |
| 126 | -Ph | $-CH_2C\equiv CH$ | -Et | -i-Bu | 83-86 | D+A |
| 128 | -Ph | $-CH_2C\equiv CH$ | -H | -OEt | 122-124 | F+ A |
| 129 | -Ph | $-CH_2C\equiv CH$ | -Et | -cyclopropyl | 110-112 | D+ A |
| 130 | -3-ClPh | $-CH_2C\equiv CH$ | -Et | $-CF_3$ | 123-125 | B |
| 131 | -Ph | $-CH_2C\equiv CH$ | -Et | $-CH=CMe_2$ | 94-97 | D+A |
| 132 | -3-BrPh | $-CH_2C\equiv CH$ | -Et | -Et | 97-99 | D+A |
| 133 | -1-oxo-4-pyridyl | $-CH_2C\equiv CH$ | -Et | $-CF_3$ | 129-131 | B+Z8 |
| 134 | -2,6-diCl-4-pyridyl | $-CH_2C\equiv CH$ | -H | $-CF_3$ | 149-152 | B |
| 135 | -2,6-diCl-4-pyridyl | $-CH_2C\equiv CH$ | -Me | -Me | 168-170 | D+A |
| 136 | -2,6-diCl-4-pyridyl | $-CH_2C\equiv CH$ | -Et | -Me | 138-140 | D+A |
| 137 | -3-CN-Ph | $-CH_2C\equiv CH$ | -Et | -Et | 115-120 | D+A+Z14+ Z16+Z17 |
| 138 | -3-(1,3-dioxolan-2-yl)-Ph | $-CH_2C\equiv CH$ | -Et | -Et | 80.5-83 | D+A |
| 139 | -3-(HON=CH)-Ph | $-CH_2C\equiv CH$ | -Et | -Et | 190-192 | D+A+Z14+ Z16 |
| 140 | -3-Cl-4-F-Ph | $-CH_2C\equiv CH$ | -Et | -Et | 80-82 | D+A |
| 141 | -2-CN-4-pyridyl | $-CH_2C\equiv CH$ | -Et | $-CF_3$ | 120-122 | B+Z8+Z9 |
| 142 | -2,6-diMeO-4-pyridyl | $-CH_2C\equiv CH$ | -Et | -Et | Oil | D+A |
| 143 | -2,6-diCl-4-pyridyl | $-CH_2C\equiv CH$ | -H | -Et | 132-134 | D+A |

| | | | | | | |
|---|---|---|---|---|---|---|
| 144 | -2-MeO-4-pyridyl | $-CH_2C{\equiv}CH$ | -Et | -Et | 81-83 | D+A |
| 145 | -2-F-4-pyridyl | $-CH_2C{\equiv}CH$ | -Et | -Et | 90-100 | D+ A |
| 146 | -2,6-diCl-4-pyridyl | $-CH_2C{\equiv}CH$ | -Me | -Et | 147-150 | D+A |
| 147 | -2,6-diCl-4-pyridyl | $-CH_2C{\equiv}CH$ | -Et | -n-Pr | 140-142 | D+A |
| 148 | -3-Cl-Ph | $-CH_2C{\equiv}CH$ | -Et | -n-Pr | 56-61 | D+A |
| 149 | -3-F-Ph | $-CH_2C{\equiv}CH$ | -Et | -n-Pr | 86-87.5 | D+ A |
| 150 | $-2-CF_3O-Ph$ | $-CH_2C{\equiv}CH$ | -Et | -Et | 55-58 | D+A |
| 151 | -2-Me-4-pyridyl | $-CH_2C{\equiv}CH$ | -Et | -Et | 73-75 | D+A |
| 152 | -Ph | $-CH_2C{\equiv}CH$ | -Et | -s-Bu | 43-47 | D+A |
| 153 | -Ph | $-CH_2C{\equiv}CH$ | -Et | $-CHF_2$ | 123-124 | B |
| 154 | -Ph | $-CH_2C{\equiv}CH$ | -Et | -n-pentyl | 35-39 | D+A |
| 155 | -3,4-diF-Ph | $-CH_2C{\equiv}CH$ | -Et | -Et | 75-79 | D+A |
| 156 | $-3-CF_3O-Ph$ | $-CH_2C{\equiv}CH$ | -Et | -Et | Oil | D+A |
| 157 | -Ph | $-CH_2C{\equiv}CH$ | -Et | -cyclopentyl | 120-123 | D+A |
| 158 | -4-pyridyl | $-CH_2C{\equiv}CH$ | -Et | -Et | 116-119 | D+A |
| 159 | -3-F-Ph | $-CH_2C{\equiv}CH$ | -Et | -Cl | 80-82 | E+H+I+ A |
| 160 | -3-pyridyl | $-CH_2C{\equiv}CH$ | -Et | -Et | 92-94 | D+A |
| 161 | -3-Cl-Ph | $-CH_2C{\equiv}CH$ | -Et | Cl | 103-106 | E+H+I+A |
| 162 | -1-oxo-4-pyridyl | $-CH_2C{\equiv}CH$ | -Et | -Et | 127-130 | D+A+Z8 |
| 163 | -3-Cl-Ph | $-CH_2C{\equiv}CH$ | -Et | -Me | 90-92 | D+A |
| 164 | -3-Cl-Ph | $-CH_2C{\equiv}CH$ | -H | -Et | 123-125 | D+A |
| 165 | -1-oxo-3-pyridyl | $-CH_2C{\equiv}CH$ | -Et | -Et | 108-111 | D+A+Z8 |
| 166 | -3,4,5-triF-Ph | $-CH_2C{\equiv}CH$ | -Et | -Et | 113-114 | D+A |
| 167 | 2-Cl-Ph | $-CH_2C{\equiv}CH$ | -Et | -Et | 91-93.5 | D+A |
| 168 | -Ph | $-CH_2C{\equiv}CH$ | -Et | $-n-C_7H_{15}$ | | |

| | | | | | | |
|---|---|---|---|---|---|---|
| | | | | | 63-66 | D+A |
| 169 | -6-Cl-2-pyridyl | $-CH_2C\equiv CH$ | -H | $-CF_3$ | 120-121 | B |
| 170 | -4-Cl-2-thienyl | $-CH_2C\equiv CH$ | -Et | -Et | 134-137 | D+A |
| 171 | -2-thienyl | $-CH_2C\equiv CH$ | -Et | -Et | 135-136.5 | D+ A |
| 172 | -Ph | $-CH_2C\equiv CH$ | -Cl | -Et | 112-114 | D+Y4+A |
| 173 | -2,6-diF-4-pyridyl | $-CH_2C\equiv CH$ | -H | $-CF_3$ | 135-137 | B |
| 174 | -5-Cl-2-thienyl | $-CH_2C\equiv CH$ | -Et | -Et | 131-133 | D+A |
| 175 | -Ph | $-CH_2C\equiv N$ | -Et | -Et | 239-240 | D+A |
| 176 | -2,6-diCl-4-pyridyl | $-CH_2C\equiv CH$ | -Me | $-CF_3$ | 131-134 | B |
| 177 | -5-Cl-3-thienyl | $-CH_2C\equiv CH$ | -Et | -Et | 134-136.5 | D+ A |
| 178 | -2,5-diCl-3-thienyl | $-CH_2C\equiv CH$ | -Et | -Et | Oil | D+A |
| 179 | -Ph | $-CH_2COCH_3$ | -Et | $-CF_3$ | 131-133 | B+Z3 |
| 180 | -5-Cl-3-pyridyl | $-CH_2C\equiv CH$ | -Et | -Et | Oil | D+A |
| 181 | -Ph | cis & trans $-CH_2CH=CHCl$ | -Et | -Et | Oil | D+A |
| 182 | -Ph | $-CH_2C\equiv CSiMe_3$ | -Et | -Et | Oil | D+A+Z11 |
| 183 | -Ph | $-CH_2C\equiv CH$ | -OMe | -Et | 131-132.5 | D+ A |
| 184 | $-3-F-5-CF_3-Ph$ | $-CH_2C\equiv CH$ | -Et | -Et | 64-66 | D+A |
| 207 | -3-furyl | $-CH_2C\equiv CH$ | -Et | -Et | 117-119 | D+A |
| 212 | -3-Cl-4-Me-Ph | $-CH_2C\equiv CH$ | -H | $-CF_3$ | 148-150 | B |
| 215 | -3,5-diCl-4-Me-Ph | $-CH_2C\equiv CH$ | -H | $-CF_3$ | 113-115 | B |
| 219 | -Ph | $-CH_2C\equiv CI$ | -Et | $-CF_3$ | 120-125 | B+Z18 |

| | | | | | | |
|---|---|---|---|---|---|---|
| 220 | -3,5-diCl-4-F-Ph | $-CH_2C{\equiv}CH$ | -H | $-CF_3$ | - | B |

* Compound 81 is a mixture with differing substituents at the $R^6$ position.

The following $^1H$-NMR data is provided for compounds in the above table which were oils or solids whose melting points were not determined. These spectra were recorded at 200 MHz in $CDCl_3$. Chemical shifts are expressed in parts per million downfield of tetramethylsilane, which was used as standard.

| Compound No. | $^1H$-NMR |
|---|---|
| 11 | 1.25(9H,s), 2.35(1H,t), 4.6(2H,d), 6.5(1H,s), 7.55(3H,m), 7.7(2H,m) |
| 18 | 4.6(2H,m), 5.0(1H,dd), 5.25(1H,dd), 5.9(1H,m), 6.9(1H,s), 7.55(5H,m) |
| 62 | 1.25(3H,t), 2.5(1H,t), 2.80(2H,q), 4.65(2H,d), 7.35(1H,s), 7.6(1H,d), 8.5(1H,d) |

| | |
|---|---|
| 67 | 1.15(3H,t), 1.2(6H,d), 2.5(1H,t), 2.65(2H,q), 3.15(1H,m), 4.6(2H,d), 7.65(2H,s) |
| 81 | 2.4(1H,t), 4.3(2H,s), 4.6(2H,d), 6.65(1H,s), 7.55(3H,m), 7.7(2H,m) [6-$CH_2Br$] |
| | 2.4(1H,t), 4.4(2H,s), 4.6(2H,d), 6.71(1H,s), 7.55(3H,m), 7.7(2H,m) [6-$CH_2Cl$] |
| 92 | 0.25(9H,s), 1.25(3H,t), 2.37(1H,t), 2.85(2H,q), 4.60(2H,d), 7.55(3H,m), 7.75(2H,m) |
| 120 | 1.25(3H,t), 2.43(1H,t), 2.61(2H,q), 4.68(2H,d), 7.5(2H,d), 7.55(3H,m), 7.75(2H,m), 8.75(2H) |
| 142 | 1.15(3H,t), 1.25(3H,t), 2.35(1H,t), 2.60(4H,q), 3.95(6H,s), 4.55(2H,d), 6.55(2H,s). |
| 156 | 1.25(6H,m), 2.4(1H,t), 2.65(4H,q), 4.6(2H,d), 7.35 - 7.75(4H,m) |
| 178 | 1.35(6H,m), 2.78(4H,m), 2.95(1H,t), 4.9(2H,d), 8.2(1H,s) |
| 180 | 1.20(3H,t), 1.25(3H,t), 2.45(1H,t), 2.65(4H,q), 4.60(2H,d), 8.10(1H,s), 8.75(1H,s), 8.85(1H,s). |
| 181 | 1.2(6H,m), 2.65(4H,m), 4.5(2H,m), 5.95(2H,m), 7.5(5H,m), (cis) 1.2(6H,m), 2.65(4H,m), 4.7(2H,m), 5.95(1H,m), 6.15(1H,m), 7.5(5H,m) (trans) |
| 182 | 0.18(9H,s), 1.25(6H,m), 2,65(4H,q), 4.6(2H,s), 7.42-7.8(5H,m) |
| 220 | 2.5(1H,t), 4.6(2H,d)6.9(1H,s), 7.8(2H,d) |

The following table of compounds are additional compounds listed as examples within the embodiment of the present invention:

TABLE 2

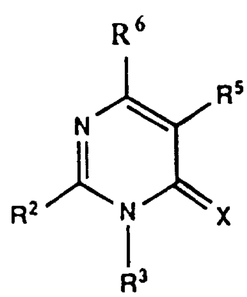

For the below table, "Me" is methyl, "Et" is ethyl, "Pr" is propyl and "Ph" is phenyl. For the compounds, X is preferably oxygen.

| No | $R^2$ | $R^3$ | $R^5$ | $R^6$ |
|---|---|---|---|---|
| 186 | -Ph | $-CH_2C{\equiv}CH$ | $-CF_3$ | -Et |
| 187 | -Ph | $-CH_2C{\equiv}CH$ | -Et | -CN |
| 188 | -Ph | $-CH_2C{\equiv}CH$ | $-CH_2F$ | -Et |
| 189 | -Ph | $-CH_2C{\equiv}CH$ | -OMe | -i-Pr |
| 190 | -Ph | $-CH_2C{\equiv}CH$ | -OMe | -n-Pr |
| 191 | -Ph | $-CH_2C{\equiv}CH$ | -OMe | -Cl |
| 192 | -2,6-diCl-4-pyridyl | $-CH_2C{\equiv}CH$ | -Et | -Cl |
| 193 | $-2-CF_3-4$-pyridyl | $-CH_2C{\equiv}CH$ | -Et | -Et |
| 194 | -4-Cl-2-pyridyl | $-CH_2C{\equiv}CH$ | -Et | -Et |
| 195 | -4,6-diCl-2-pyridyl | $-CH_2C{\equiv}CH$ | -Et | -Et |
| 196 | -2-pyridyl | $-CH_2C{\equiv}CH$ | -Et | -Et |
| 197 | -2-naphthyl | $-CH_2C{\equiv}CH$ | -Et | -Et |
| 198 | -2,6-diF-4-pyridyl | $-CH_2C{\equiv}CH$ | -Et | -Et |
| 199 | -2,6-diCl-4-pyridyl | $-CH_2C{\equiv}CH$ | -OMe | -Et |
| 200 | -Ph | $-CH_2C{\equiv}CH$ | -Et | $-C(CH_3)=CH_2$ |
| 201 | -Ph | $-CH_2C{\equiv}CH$ | -Et | $-CH_2C{\equiv}CH$ |
| 202 | -Ph | $-CH_2C{\equiv}CH$ | -Et | $-CH_2CH=CH_2$ |
| 203 | -Ph | $-CH_2C{\equiv}CH$ | -Et | -OPh |
| 204 | -Ph | $-CH_2C{\equiv}CH$ | -Et | -OMe |
| 205 | -Ph | $-CH_2C{\equiv}CH$ | -Et | $-OCHF_2$ |
| 206 | -Ph | $-CH_2C{\equiv}CH$ | $-OCHF_2$ | -Et |
| 208 | -5-Cl-3-furyl | $-CH_2C{\equiv}CH$ | -Et | -Et |
| 209 | -3-F-Ph | $-CH_2C{\equiv}CH$ | -H | $-CF_3$ |

| | | | | |
|---|---|---|---|---|
| 210 | -3,5-diCl-Ph | $-CH_2C\equiv CH$ | -H | $-CF_3$ |
| 211 | -3,5-diF-Ph | $-CH_2C\equiv CH$ | -H | $-CF_3$ |
| 213 | -3-Cl-4-F-Ph | $-CH_2C\equiv CH$ | -H | $-CF_3$ |
| 214 | -3,4-diF-Ph | $-CH_2C\equiv CH$ | -H | $-CF_3$ |
| 216 | -3,4-methylenedioxy-Ph | $-CH_2C\equiv CH$ | -Et | -Et |
| 217 | -5-Cl-3-thienyl | $-CH_2C\equiv CH$ | -Et | $-CF_3$ |
| 218 | -3,4,5-triF-Ph | $-CH_2C\equiv CH$ | -Et | $-CF_3$ |

Methods of Preparation.

The 2-substituted pyrimidines of the present invention may be prepared by standard synthetic routes such as those illustrated below.

Method A - General Description:

A precursor compound having the structure of formula I above with hydrogen (H) in the $R^3$ substituent position is selected. Reaction with $R^3Y$ is performed in a base-solvent mixture. Y can be a halogen, alkanesulfonate, haloalkanesulfonate or optionally substituted benzenesulfonate. The bases can be sodium hydride, alkali metal hydroxides, alkali metal carbonates or alkali metal alkoxides. The solvent can be alcohol, ketone, water, ether, DMSO or DMF. A mixture of N- and O- alkylated products results.

Method A - Specific Example 1 -Preparation of 6-ethyl-5-methvl-2-phenyl-3-propargyl-4(3H)-pyrimidinone (Compound 5):

To a stirred solution of 24.30g (0.45 mol) of sodium methoxide in 400 mL of methanol was added a slurry of 61.78g (0.29 mol) of 6-ethyl-5-methyl-2-phenyl-4(3H)-pyrimidinone in 100 mL of methanol. The mixture was heated to reflux to give a clear orange solution to which 44.89g (0.30 mol) of an 80% weight solution of propargyl bromide in toluene was added. The course of the reaction was followed by GC. Refluxing was continued for 6.5h, when an additional 20.63g (0.14mol) of an 80% weight solution of propargyl bromide in toluene was added. Refluxing was continued for an additional 4.5h. The reaction mixture was allowed to cool to room temperature and 250 mL of water and 250 mL of saturated aqueous $NaHCO_3$ were added. The mixture was rotovaped to remove the bulk of the methanol and extracted with three 200 mL portions of ethyl acetate. The ethyl acetate extracts were combined, filtered and extracted with three 200 mL portions of 5% aq HCl. The combined aqueous HCl extracts were basified to pH 11 with 50% aqueous NaOH and extracted with three 250mL portions of ether. The combined ether extracts were washed with 100mL of brine, dried over $MgSO_4$ and rotovaped to leave 30.46g of a yellowish solid. This material was triturated with 100mL portions of 10, 25 and 50% ether in hexanes and recrystallized from 25mL of toluene to furnish 15.11 g. of Compound 5 as a white solid mp 115-117°C. $^1H$-NMR ($d^6$-DMSO) $\delta$ 1.15(3H, t, J=7.8),
2.07(3H, s), 2.58(2H, q, J=7.8), 3.32(1H, t, J=2.4), 4.55(2H, d, J=2.4), 7.58(3H, m), 7.65(2H, m).

Method B - General Description:

Direct condensation of an N-alkylamidine and a beta-keto ester is performed by warming the reagents in a solvent such as THF or neat:

$$R^2C(=NH)N(H)R^3 + R^6C(=O)CH(R^5)C(=O)OR \rightarrow \text{2-substituted pyrimidine (Figure I)}$$

Preferably $R^3$ is a non-reactive group and when $R^3$ is a propargyl group, preferably $R^6$ is $CF_3$.

Method B - Specific Example - Preparation of 5-ethyl-2-phenyl-3-propargyl-6-trifluoromethyl-4(3H)-pyrimidinone

To a stirred solution of 22.66g (0.13mol) of methyl benzimidate hydrochloride in 80mL of methanol was added 11.09g (0.13mol) of powdered sodium bicarbonate. The mixture was stirred for 0.5h and 9.1mL (0.13mol) of propargylamine was added. The mixture was stirred for 4h at room temperature and then rotovapped to remove most of the methanol. To the oily orange residue was added 34.00g of 80% pure ethyl 2-trifluoroacetylbutyrate (0.13mol). The mixture was heated at 55 - 60°C for 40h. The mixture was diluted with 300mL of ether, washed with two 150mL portions of 5% aqueous HCl and 150mL of saturated aqueous sodium bicarbonate and dried over $MgSO_4$. Removal of the solvent and drying at 50°C under high vacuum afforded 22.58g of an orange solid. This material was purified by flash chromatography on 325g of silica gel, eluting with 0, 10, 20, 30 and finally 40% ether in hexanes to furnish 16.86g of a yellow solid. This material was triturated with two 100mL portions of boiling hexanes and recrystallized from 150mL of 10% ether in hexanes to give 10.31g (26%) of 5-ethyl-2-phenyl-3-propargyl-6-trifluoromethyl-4(3H)-pyrimidinone (Compound No. 46) as a white solid, mp 111 - 114°C. $^1$H-NMR ($CDCl_3$) $\delta$ 1.24(3H,t), 2.38(1H,t), 2.75(2H,q), 4.62(2H, d), 7.55(3H,m), 7.74(2H,m).

Method D - General Description

An amidine hydrochloride or other salt is heated with a beta-keto ester in a solvent in the presence of a base to neutralize the hydrochloric acid. Solvents usable include xylene or toluene, preferably, or ethanol or heptane. Sodium acetate or sodium ethoxide can be the base:

$$R^2C(=NH)NH_2 + R^6C(=O)CH(R^5)C(=O)OR \rightarrow \text{Figure I}$$

with $R^3$ = H; precursor for Method A

Method D - Preparation of 6-ethyl-5-methyl-2-phenyl-4(3H)-pyrimidinone.

A 2L 3-neck flask equipped with mechanical stirrer, Dean Stark trap and a reflux condenser was purged with nitrogen and charged with 93.91 g. (0.59 mol) of methyl 2-propionylpropionate, 103.58g (≤0.66mol) of benzamidine hydrochloride hydrate, 54.30g (0.66mol) of anhydrous sodium acetate and 1L of xylenes. The mixture was stirred and heated to reflux under a nitrogen atmosphere for 46 h.

The Dean Stark trap was replaced with a distillation head and about 75% of the solvent was distilled off at atmospheric pressure. After the flask had cooled to room temperature, 500mL of water and 200mL of ether were added. The mixture was filtered and the solid collected was washed thoroughly with water and ether and dried in a vacuum oven at 60°C to give 74.73g (50%) of the desired product, mp 195-199°C. $^1$H-NMR ($d^6$-DMSO) $\delta$ 1.23(3H, t, J=7.8), 2.02 (3H, s), 2.61(2H, q, J=7.8), 7.50 (3H,m), 8.13(2H, m).

The filtrate was separated into aqueous and organic layers. The organic layer was extracted with three 125mL portions of 5% aqueous NaOH. The combined aqueous base extracts were acidified to pH 7 with aqueous HCl, allowed to cool and filtered. The solid collected was washed with water and ether and dried in a vacuum oven at 60°C to afford an additional 5.16g (4%) of the desired product, mp 196-199°C.

Method E - General Description

Malonate diester is condensed with an amidine under basic conditions. For example, sodium methoxide in refluxing methanol may be used:

$$R^2C(=NH)NH_2 + ROC(=O)CH(R^5)C(=O)OR \rightarrow \text{Figure I}$$

with $R^3$ = H and $R^6$ = OH.

Method E - Preparation of 5-ethyl-6-hydroxy-2-phenyl-4(3H)-pyrimidinone

A mixture of 45.19g (0.29mol) of benzamidine hydrochloride hydrate, 127.42g (0.59mol) of 25% sodium methoxide in methanol, 55mL (0.29mol) of diethyl ethylmalonate and 175mL of methanol was heated at reflux for 25h. The mixture was rotovapped to remove the bulk of the methanol. The residue was diluted with 300mL of water and the pH was adjusted to 7 with concentrated hydrochloric acid. The solid precipitate was collected by filtration and dried under

vacuum at 50°C to afford 31.89g (51%) of crude 5-ethyl-6-hydroxy-2-phenyl-4(3H)-pyrimidinone as a pale yellow solid. $^1$H-NMR (d6-DMSO) δ 1.05(3H,t), 2.39(2H,q), 7.5(3H,m), 8.1(2H,m).

Method F - General Description

Method F is similar to Method D except that a 3-alkoxyacrylate ester is used instead of a beta-keto ester :

$$R^2C(=NH)NH_2 + RO(R^6)C=C(R^5)C(=O)OR \rightarrow \text{Figure I}$$

with $R^3$ = H
Various conditions are usable : for example, amidine hydrochloride/ 3-alkoxyacrylate in NaOAc/DMSO at 120°C or in sodium methoxide/ethanol at 5°C.

Method F - Specific Example - Preparation of 6-ethoxy-2-phenyl-4(3H)-pyrimidinone

A mixture of 3.14g (20.0mmol) of benzamidine hydrochloride hydrate, 1.65g (20.lmmol) of powdered anhydrous sodium acetate, 4.17g (22.2mmol) of ethyl 3,3-diethoxyacrylate and 10mL of DMSO was heated at 120°C for 8h. The mixture was cooled, diluted with 50mL of 5% aqueous NaOH and washed with two 100mL portions of ether. The aqueous layer was acidified with concentrated hydrochloric acid and the precipitate was collected by filtration and dried under vacuum at 50°C to furnish 2.28g (57%) of crude 6-ethoxy-2-phenyl-4(3H)-pyrimidinone as a yellow solid $^1$H-NMR (d6-DMSO)δ 1.35(3H,t), 4.33(2H,q), 5.60(1H,s), 7.50(3H,m), 8.2(2H,m).

Method H - General Description

6-Hydroxy-4(3H)-pyrimidinones were heated with phosphorus oxyhalide with or without a cosolvent to give 6-halo-4(3H)-pyrimidinones. For example, phosphorous oxybromide was used with an inert solvent (1,2-dichloroethane) at reflux. See U. S. Patent 4,617,393.

Method H - Specific Example - Preparation of 4,6-dibromo-5-ethyl-2-phenylpyrimidine

A mixture of 24.50g (85.3mmol) of phosphorus oxybromide, 7.56g (37.3mmol) of 5-ethyl-6-hydroxy-2-phenyl-4 (3H)-pyrimidinone and 20mL of 1,2-dichloroethane was heated at reflux for 2h. After cooling to room temperature, the mixture was poured onto 300g of crushed ice. The ice was allowed to melt, the mixture was basified by cautious addition of solid $Na_2CO_3$ and then extracted with two 200mL portions of ethyl acetate. The combined ethyl acetate extracts were dried over $MgSO_4$ and concentrated to afford 11.79g (92%) of crude 4,6-dibromo-5-ethyl- 2-phenylpyrimidine. This material was recrystallized from hexanes to furnish 6.62g (52%) of pure product, mp 101 - 103°C. $^1$H-NMR ($CDCl_3$) δ 1.20(3H,t), 2.95(2H,q), 7.45(3H,m), 8.35(2H,m).

Method I - General Description

4,6-dihalopyrimidines are acidically hydrolyzed to give 6-halo-4(3H)-pyrimidinones. See U. S. Patent 4,617,393.

Method I - Specific Example - Preparation of 6-bromo-5-ethyl-2-phenyl-4(3H)-pyrimidinone

To 8.29g (26.1 mmol) of crude 4,6-dibromo-5-ethyl-2-phenylpyrimidine was added a mixture of 4mL of water and 15mL of concentrated sulfuric acid. The mixture was stirred for 18h and poured onto 200g of crushed ice. After the ice had melted the precipitate was collected by filtration and dried under vacuum to afford 7.21g (99%) of crude 6-bromo-5-ethyl-2-phenyl-4(3H)-pyrimidinone. $^1$H-NMR (d6-DMSO) δ 1.10(3H,t), 2.55(2H,q), 7.55(3H,m), 8.10(2H,m).

Method Y1

(a) Preparation of 5-bromo-2-phenyl-6-trifluoromethyl-4(3H)-pyrimidinone.

To a solution of 1.0 g (3.94 mmol) of 2-phenyl-6-trifluoromethyl-4(3H)-pyrimidinone and 20 mL of glacial acetic acid was added 1.0 g (5.6 mmol) N-bromosuccinimide and the mixture was left to stir at room temperature for 16h. The reaction was poured onto ice water and vacuum filtered, washing well with water. The crude product was recrystallized from ethyl acetate to yield 1.05g (83.5%) of 5-bromo-2-phenyl-6-trifluoromethyl-4(3H)-pyrimidinone, as a white

solid. $^{1}$H-NMR ($d_6$DMSO) δ 7.6 (3H, m); 8.15 (2H, m).

(b) Preparation of 5-bromo-2,6-diphenyl-4(3H)-pyrimidinone.

To a suspension of 13.37 g (56 mmol) of 2,6-diphenyl-4(3H)-pyrimidinone and 200 mL glacial acetic acid was added 15.1 g (84.8 mmol) of N-bromosuccinimide and the mixture was left to stir at room temperature for 60h. The reaction was poured onto 100 g crushed ice and vacuum filtered, washing well with water, then air dried to yield 8.85 g (48%) 5-bromo-2,6-diphenyl-4(3H)-pyrimidinone, as a white solid. $^{1}$H-NMR ($d_6$DMSO) δ 7.55 (6H, m); 7.75 (2H, m); 8.15 (2H, m).

Method Y2 - Preparation of 6-ethyl-5-iodo-2-phenyl-4(3H)-pyrimidinone

A mixture of 8.18g (40.9mmol) of 6-ethyl-2-phenyl-4(3H)-pyrimidinone, 1.68g (42.0mmol) of sodium hydroxide, 10.42g (41.0mmol) of iodine and 50mL of water was heated at 50°C for 4h. The mixture was cooled and filtered. The white solid collected was dried in a vacuum oven to leave 12.60g (75%) of 6-ethyl-5-iodo-2-phenyl-4(3H)-pyrimidinone. $^{1}$H-NMR (d6-DMSO) δ 1.25(3H,t), 2.85(2H,q), 7.50(3H,m), 8.15(2H,m).

Method Y3 - Preparation of 4,6-difluoro-5-ethyl-2-phenyl-pyrimidinone.

To a stirred solution of 3.14g (12.41mmol) portion of 4,6-dichloro-5-ethyl-2-phenylpyrimidine in 25mL of sulfolane at 70 - 80°C was added 6.37g (109.8mmol) of spray-dried potassium fluoride. The mixture was heated at 200°C for 0.5h. After cooling, the mixture was diluted with 100mL of water and extracted with 400mL of 1:1 ether:hexanes. The organic layer was washed with two 100mL portions of water, dried over $MgSO_4$ and concentrated to give 2.30g of crude product. This material was combined with 0.29g of crude product from another run and purified by flash chromatography on a column of 40g of silica gel. The column was eluted with 0, 5, 10, 15 and 20% ether in hexanes to furnish 2.18g (71%) of 4,6-difluoro-5-ethyl-2-phenylpyrimidine as a white solid, m.p. 49 - 51°C. $^{1}$H-NMR ($CDCl_3$) δ 1.2(3H,t), 2.65 (2H,q), 7.50(3H,m), 8.4(2H,m).

Method Y4 - Preparation of 5-Chloro-6-ethyl-2-phenyl-4(3H)-pyrimidinone

A stirred solution of 7.81g (39.1mmol) of 6-ethyl-2-phenyl-4(3H)-pyrimidinone and 5.80g (43.4mmol) of N-chlorosuccinimide in 100mL of glacial acetic acid was heated at 90°C for 4h. The mixture was cooled, poured onto crushed ice and allowed to stand until the ice had melted. The mixture was filtered and the solid collected was washed with water and a little ether. The solid was dried in a vacuum oven at 50C to afford 7.99g of 5-chloro-6-ethyl-2-phenyl-4 (3H)-pyrimidinone (an intermediate for compound 172) as a white solid. $^{1}$H-NMR (d6-DMSO) 1.30(3H,t), 2.8(2H,q), 7.6(3H,m), 8.2(2H.m).

Method Z1 - Preparation of 6-dimethylaminocarbonyl-5-methyl-2-phenyl-3-propargyl-4(3H)-pyrimidinone. (Compound 19)

To a solution of 1.81g (6.1mmol) of 6-ethoxycarbonyl-5-methyl-2-phenyl-3-propargyl-4(3H)-pyrimidinone in 100mL of ethanol and 50mL of THF was added 50mL of 5% aqueous sodium hydroxide. The mixture was stirred at room temperature for 24h and rotovapped to remove the bulk of the organic solvents. The residue was diluted with 50mL of 5% aqueous sodium hydroxide and washed with 100mL of ether. The aqueous phase was acidified with concentrated hydrochloric acid and extracted with two 100mL portions of ethyl acetate. The combined ethyl acetate extracts were washed with 50mL of brine, dried over $MgSO_4$ and concentrated to leave 0.87g (53%) of crude 6-carboxy-5-methyl-2-phenyl-3-propargyl-4(3H)-pyrimidinone as a brown oil.

To a stirred solution of 0.87g (3.2mmol) of crude 6-carboxy-5-methyl-2-phenyl-3-propargyl-4(3H)-pyrimidinone, 0.32g (3.9mmol) of dimethylamine hydrochloride and 2mL of pyridine in 10mL of THF was added 0.74g (3.6mmol) of solid N,N'-dicyclohexylcarbodiimide. The mixture was stirred at room temperature for 4 days and filtered to remove insoluble material. The filtrate was diluted with 150mL of ethyl acetate, washed with 50mL of 5% aqueous HCl and 50mL of saturated aqueous sodium bicarbonate and dried over $MgSO_4$. Removal of the solvent left 0.40g of crude product which was purified by flash chromatography on a 30g column of silica gel, eluted with 60, 80 and 100% ethyl acetate in hexanes to furnish 0.30g (32%) of 6-dimethylaminocarbonyl-5-methyl-2-phenyl-3-propargyl-4(3H)-pyrimidinone (compound 19), mp 137 - 140°C. $^{1}$H-NMR ($CDCl_3$) δ 2.15(3H,s), 2.40(1H,t), 3.00(3H,s), 3.10(3H,s), 4.65(2H,d), 7.55(3H,m), 7.70 (2H,m).

Method Z2 - Preparation of 6-dimethylamino-5-methyl-2-phenyl-3-propargyl-4(3H)-pyrimidinone. (Compound 23)

To an ice cooled solution of 1.5 g (3.8 mmol) 6-chloro-5-methyl-2-phenyl-3-propargyl-4(3H)-pyrimidinone in 4 mL of tetrahydrofuran, was added 22 mL (99 mmol) of 4.5 M dimethylamine in ether portionwise (2-4 mL) over a period of 7 days. The reaction mixture was allowed to warm and stir at room temperature after each addition. The progress of the reaction was followed by gas chromatography and proceeded to 80% completion. The solvent was removed *in vacuo* and the residue was taken up in ether and washed twice with water. The organic layer was dried over $MgSO_4$ and concentrated to yield 1.15 g crude solid product. Flash column chromatography on silica gel (gradient elution 25-30% ethyl acetatehexane) afforded pure 6-dimethylamino-5-methyl-2-phenyl-3-propargyl-4(3H)-pyrimidinone (compound 23), as a white solid. $^1$H-NMR ($CDCl_3$) δ 2.2 (3H, s); 2.35 (1H, t); 3.5 (6H, s); 4.6 (2H, d); 7.65 (3H, m); 7.75 (2H, m).

Method Z3 - Preparation of 5-Ethyl-3-(2-oxopropyl)-2-phenvl-6-trifluoromethyl-4(3H)-pyrimidinone (Compound 179)

To a stirred solution of 4.83g (15.8mmol) of 5-ethyl-3-propargyl-2-phenyl-6-trifluoromethyl-4(3H)-pyrimidinone (compound 46) in 50mL of THF was added 50mL of 10% aq NaOH. The mixture was heated at reflux for 2h, cooled and diluted with 150mL of ethyl acetate. The organic layer was separated, washed with 50mL of water and 50mL of brine and dried over $MgSO_4$. Removal of the solvent afforded 4.74g of 5-ethyl-3-(2-oxopropyl)-2-phenyl-6-trifluoromethyl-4(3H)-pyrimidinone (compound 179) as a white solid. $^1$H-NMR ($CDCl_3$) 1.2(3H), 2.2(3H,s), 2.7(2H,q), 4.7(2H,s), 7.45(5H,m).

Method Z4 - Preparation of 5-methyl-2-phenyl-3-propargyl-6-methylthio-4(3H)

pyrimidinone. (Compound 26)

To a solution of 2.5 g (9.67 mmol) of 6-chloro-5-methyl-2-phenyl-3-propargyl-4(3H)-pyrimidinone in 100 mL of methanol, was added 0.8 g (11.4 mmol) sodium thiomethoxide and the reaction was stirred at room temperature for 4 days. The methanol was evaporated and the residue was dissolved in 100 mL of ethyl acetate, then washed three times with 50 mL of 1 M sodium hydroxide followed by one time with 50 mL of brine. The organic layer was dried over $MgSO_4$ and concentrated to yield 2.6 g crude product. Flash column chromatography on silica gel (100% methylene chloride) afforded 5-methyl-2-phenyl-3-propargyl-6-thiomethyl-4(3H)-pyrimidinone (Compound 26) as a white solid. $^1$H-NMR ($CDCl_3$) δ 2.1 (3H, s); 2.35 (1H, t); 2.5 (3H, s); 4.55 (2H, d); 7.5 (3H, m); 7.7 (2H, m).

Method Z5 - Preparation of 3-(2,2-dimethoxypropyl)-6-ethyl-5-methyl-2-phenyl-4(3H)-pyrimidinone (Compound 36)

To a stirred suspension of 4.51g (17.9mmol) of 6-ethyl-5-methyl-2-phenyl-3-propargyl-4(3H)-pyrimidinone (Compound 5) in 30mL of methanol was added 7.50g (34.7mmol) of a 25% by weight solution of sodium methoxide in methanol. The mixture was warmed until homogeneous and 2.2mL (35.3mmol) of methyl iodide was added. The mixture was refluxed for 4h and then rotovapped to remove the bulk of the methanol. The residue was partitioned between 100mL of water and two 100mL portions of ether. The combined ether layers were washed with 50mL of brine and dried over $MgSO_4$. Removal of the solvent afforded 4.35g of a yellow oil. Flash chromatography on a column of 50g of silica gel, eluting with 20, 30, 40 and 50% ether in hexanes furnished 3.30g (58%) of 3-(2,2-dimethoxypropyl)-6-ethyl-5-methyl-2-phenyl-4(3H)-pyrimidinone (compound 36), mp 80 - 83°C. $^1$H-NMR ($CDCl_3$) δ 1.15(3H,s), 1.25(3H,t), 2.15 (3H,s), 2.65(2H,q), 2.85(6H,s), 4.4(2H,m), 7.45(5H,s).

Method Z6 - Preparation of 3-methoxymethyl-2-phenyl-6-trifluoromethyl-4(3H)-pyrimidinone (Compound 6)

To a solution of 1.5 g (5.9 mmol) of 2-phenyl-6-trifluoromethyl-4(3H)-pyrimidinone, 17.2 g (226.3 mmol) dimethoxymethane, and 35 mL of chloroform was added 2.5 g (17.6 mmol) phosphorous pentoxide, at room temperature. By TLC (25% ethyl acetate in hexane) the reaction was incomplete after 4h and an additional 3 g (21.1 mmol) phosphorous pentoxide was added. Stirring was continued for 16h. The reaction mixture was poured onto crushed ice and 1 M sodium hydroxide and methylene chloride were added. The layers were separated and the aqueous layer was extracted twice with methylene chloride. The organic extracts were combined and washed with brine, then dried over $MgSO_4$ and concentrated to yield 1.1 g crude product, which was purified by recrystallization from hexane. Thus, 0.55 g (32%) 3-methoxymethyl-2-phenyl-6-trifluoromethyl-4(3H)-pyrimidinone (Compound 6) as a yellow solid was obtained. $^1$H-NMR ($CDCl_3$) δ 3.55 (3H, s); 5.2 (2H, d); 6.85 (1H, s); 7.65 (3H, m); 7.75 (2H, m).

Method Z7 - Preparation of 6-ethyl-2-phenyl-3-propargyl-5-(2-trimethylsilylethynyl)-4(3H)-pyrimidinone (Compound 92)

To a stirred solution of 3.59g (9.86mmol) of 6-ethyl-5-iodo-2-phenyl-3-propargyl-4(3H)-pyrimidinone and 16.45g (167.5mmol) of trimethylsilylacetylene in 40 mL of DMF were added 1.13g (0.98mmol) of tetrakis (triphenylphosphine) palladium(0), 0.41g (2.15mmol) of copper (I) iodide and 2.8mL (20.0mmol) of triethylamine. The mixture was stirred at room temperature for 18h, diluted with 200mL of water and extracted with two 200mL portions of ether. The combined ether extracts were dried over $MgSO_4$ and evaporated under reduced pressure to leave 5.71g of a black oil. This material was subjected to flash chromatography on a column of 50g of silica gel, eluted with 0, 10, 20, 30, 40, 50, 60 and 80% ether in hexanes to afford 0.80g of material. Further purification was effected by chromatography on a column of activity I alumina eluted with 0, 10, 20, 35, 50, 75 and 100% ether in hexanes. This process yielded 0.43g (13%) of 6-ethyl-2-phenyl-3-propargyl-5-(2-trimethylsilylethynyl)-4(3H)-pyrimidinone as an oil. IH-NMR (CDCl3) $\delta$ 0.25(9H,s), 1.25(3H,t), 2.37(1H,t), 2.85(2H,q), 4.60(2H,d), 7.55(3H,m), 7.75(2H,m).

Method Z8 - 5-Ethyl-2-(1-oxo-4-pyridyl)-3-propargyl-6-trifluoromethyl-4(3H)-pyrimidinone (Compound 133)

To a stirred suspension of 8.54g (27.8mmol) of -5-ethyl-3-propargyl-2-(4-pyridyl)-6-trifluoromethyl-4(3H)-pyrimidinone (compound 58) in 50mL of ethanol was adsded 9.07g (18.3mmol) of monoperoxyphthalic acid magnesium salt hexahydrate. The mixture was stirred at room temperature for 24h. The bulk of the ethanol was removed on the rotovap and the residue was partitioned between 150mL of ethyl acetate and 75mL of 5% aqueous hydrochloric acid. The organic layer was washed with two 75mL portions of saturated aqueous $NaHCO_3$, dried $MgSO_4$ and concentrated to leave 8.42g of 5-ethyl-2-(1-oxo-4-pyridyl)-3-propargyl-6-trifluoromethyl-4(3H)-pyrimidinone (compound 133) as a yellow solid. $^1H$-NMR ($CDCl_3$) 1.25(3H,t), 1.30(3H,t), 2.60(1H,t), 2.8(4H,m), 4.7(2H,d), 7.8(2H,d), 8.35(2H,d).

Method Z9 - Preparation of 2-(2-Cyano-4-pyridyl)-5-ethyl-3-propargyl-6-trifluoromethyl-4(3H)-pyrimidinone (Compound 141)

To a stirred solution of 6.96g (21.6mmol) of 5-ethyl-2-(1-oxo-4-pyridyl)-3-propargyl-6-trifluoromethyl-4(3H)-pyrimidinone and 6.0mL (42.8mmol) of triethylamine in 20mL of acetonitrile was added 11.5mL (86.3mmol) of trimethylsilyl cyanide. The mixture was heated at reflux for 4h. After standing overnight, the mixture was diluted with 150mL of ether, washed with three 50mL portions of water and dried over $MgSO_4$. Removal of the solvent left 4.94g of crude product as a black tar. This material was purified by flash chromatography on 60g of silica gel, eluting with 0, 20, 35, 50, 65, 80 and 100% ether in hexanes to furnish 1.78g of 2-(2-cyano-4-pyridyl)-5-ethyl-3-propargyl-6-trifluoromethyl-4(3H)-pyrimidinone (compound 141) as a solid. $^1H$-NMR ($CDCl_3$) 1.25(3H,t), 2.60(1H,t), 2.8(2H,q), 4.6(2H,d), 8.0(1H,d), 8.10 (1H,s), 9.0(1H,d).

Method Z11 - Preparation of 5,6-Diethyl-2-phenyl-3-(3-trimethylsilylprop2-ynyl)-4(3H)-pyrimidinone (Compound 182)

To an oven-dried 50mL 3-neck flask were charged 0.9g (3.38mmol) of 5,6-diethyl-2-phenyl-3-propargyl-4(3H)-pyrimidinone and 25mL freshly distilled THF. The solution was cooled to -70°C and 2.2mL of 1.6 M (3.52mmol) $\eta$-butyllithium in hexane was added at a rate to maintain the temperature below -62C during the addition. The reaction mixture turned black and was allowed to stir for 12 minutes at -70°C. A 0.47mL (3.70mmol) portion of trimethylsilyl chloride was added and the reaction stirred for 20 minutes at -70°C. The dry ice bath was removed and the reaction was left to stir and warm to room temperature overnight. The THF was removed *in vacuo* and ether was added. The ether solution was washed 3 times with water then dried over $MgSO_4$ and concentrated to yield 1.2g of crude product, as a brown oil. The crude product was purified by chromatography on a 30g silica gel column eluting with 18% ethyl acetate in hexane. 0.8g (70% yield) of 5,6-diethyl-2-phenyl-3-(3-trimethylsilyl-2-propynyl)-4(3H)-pyrimidinone (compound 182), was obtained as a yellow oil. $^1H$-NMR ($CDCl_3$) 0.18(9H,s), 1.25(6H,m), 2,65(4H,q), 4.6(2H,s), 7.6(3H,m), 7.42-7.8(5H,m)

Method Z12 - Preparation of 5-ethvl-3-(pent-2-vn-4-en-1-yl)-2-phenyl-6-trifluoromethyl-4(3H)-pyrimidinone (Compound 111)

To a deoxygenated solution of 1.01g (3.28mmol) of 5-ethyl-2-phenyl-3-propargyl-6-trifluoromethyl-4(3H)-pyrimidinone and 0.61g (3.96mmol) of vinyl iodide in 25mL of triethylamine was added a mixture of 60mg of copper (I) iodide and 60mg of bis(triphenylphosphine) palladium (II) chloride. The mixture was stirred at room temperature for 22h and rotovapped to remove the bulk of the triethylamine. The residue was taken up in 150mL of ethyl acetate, washed with 75mL of 5% aqueous hydrochloric acid, 75mL of saturated aqueous sodium bicarbonate and 75mL of brine, and dried.

Removal of the solvent left 1.51g of a brown tar. Flash chromatography on a column of 30g of silica gel eluting with 20, 40, 60 and 60% ether in hexanes afforded 0.31g of crude 5-ethyl-3-(pent-2-yn-4-en-1-yl)-2-phenyl-6-trifluoromethyl-4(3H)-pyrimidinone. A second chromatography yielded 0.25g (23%) of pure 5-ethyl-3-(pent-2-yn-4-en-1-yl)-2-phenyl-6-trifluoromethyl-4(3H)-pyrimidinone (compound 111) as a solid, mp 104 - 106°C. $^1$H-NMR ($CDCl_3$) d 1.25(3H,t), 2.8 (2H,q), 4.75(2H,s), 5.5-5.9(3H,m), 7.55(3H,m), 7.7(2H,m).

Z13 - Preparation of 2-(1-methyl-3-pyridinium)-5-ethyl-3-propargyl-6-trifluoromethyl-4(3H)-pyrimidinone iodide (Compound 61)

A solution of 1.23g (4.01mmol) of 5-ethyl-3-propargyl-2-(3-pyridyl)-6-trifluoromethyl-4(3H)-pyrimidinone and 1.0mL (16.1mmol) of methyl iodide in 5mL of $CHCl_3$ was heated at reflux for 6h. An additional 1.0mL portion of methyl iodide was added and refluxing was continued overnight. The mixture was rotovapped to leave 1.86g of 2-(1-methyl-3-pyridinium)-5-ethyl-3-propargyl-6-trifluoromethyl-4(3H)-pyrimidinone iodide as a brown solid. $^1$H-NMR ($CDCl_3$) $\delta$ 1.2 (3H,t), 2.7(1H,t), 2.75(2H,q), 4.65(3H,s), 4.9(2H,d), 8.4(1H,t), 8.9(1H,d), 9.2(1H,s), 9.5(1H,d).

Method Z14- Preparation of 5,6-diethyl-2-(3-formylphenyl)-3-propargyl-4(3H)-pyrimidinone (Compound 69)

To a solution of 2.3g (6.8mmol) of 5,6-diethyl-2-[3-(2-dioxolanyl)phenyl]-3-propargyl-4(3H)-pyrimidinone in 1mL of ethyl acetate was added 50mL of 6M hydrochloric acid and the mixture was stirred for 4 hours. The reaction was followed by gas chromatography and TLC(20% ethyl acetate in hexane). Upon completion of reaction, 75mL of ether and 150 mL of water were added to the reaction mixture. The layers were separated and the aqueous layer was extracted twice with 50 mL of ether. The organic layers were combined, dried over $MgSO_4$ and concentrated to yield 1.73g of 5,6-diethyl-2-(3-formylphenyl)-3-propargyl-4(3H)-pyrimidinone (Compound 69) as a yellow oil (86%), which solidified on standing. Mp.= 82-86°C. $^1$H-NMR ($CDCl_3$) $\delta$ 1.25(6H,m), 2.4(1H,t), 2.65(4H,q), 4.6(2H,d), 7.7(1H,t), 8.05 (2H,m), 8.25(1H,s), 10.15(1H,s).

Method Z16 - Preparation of 5,6-Diethyl-2-(3-hydroxyiminophenyl)-3-propargyl-4(3H)-pyrimidinone (Compound 139)

To a 100mL RBF were charged 1.1g (3.7mmol) of 5,6-diethyl-2-(3-formyl-phenyl)-3-propargyl-4(3H)-pyrimidinone, 0.52g (7.5mmol) of hydroxylamine hydrochloride and 50mL of ethanol. The reaction mixture was refluxed for 17 hours. The ethanol was removed *in vacuo* and ether and ethyl acetate were added to the residue. The organics were washed 3 times with water. The organic layer was gravity filtered to remove 0.22g of 5,6-diethyl-2-(3-hydroxyiminophenyl)-3-propargyl-4(3H)-pyrimidinone (compound 139). The organic layer was dried over MgSO4 and concentrated to yield a further 0.67g of 5,6-diethyl-2-(3-hydroxyiminophenyl)-3-propargyl-4(3H)-pyrimidinone (compound 139) as a white solid. A combined yield of 77.6% was obtained. $^1$H-NMR ($CDCl_3$) 1.25(6H,m); 2.35(1H,t); 2.65(4H,m); 4.6(2H,d); 7.49-8.15(4H,m); 8.7(1H,s)

Method Z17 - Preparation of 5,6-Diethyl-2-(3-cyanophenyl)-3-propargyl-4(3H) pyrimidinone (Compound 137)

To an ice cooled solution of 0.64g (2.07mmol) of 5,6-diethyl-2-(3-hydroxyiminophenyl)-3-propargyl-4(3H)-pyrimidinone in 10mL methylene chloride, 1.5mL (20.5mmol) of thionyl chloride was added dropwise. The ice bath was removed and the reaction continued to stir at room temperature for 16h. The reaction mixture was concentrated and 10mL portions of methylene chloride were added and removed *in vacuo* twice. 0.65g of a light brown solid was obtained as crude product. This was combined with 0.15g crude product from a previous run. The crude product was purified by passing it through a 4 inch plug of basic alumina and washing with 700mL of methylene chloride. 400mg of 5,6-diethyl-2-(3-cyanophenyl)-3-propargyl-4(3H)-pyrimidinone (compound 137) was obtained. $^1$H-NMR ($CDCl_3$) 1.25(6H, m); 2.4(1H,t); 2.65(4H,m); 4.58(2H,d); 7.64-8.1(4H,m).

Method Z18 - Preparation of 5-ethyl-3-(3-iodopropargyl)-2-phenyl-6-trifluoromethyl-4(3H)-pyrimidinone (Compound 219)

A stirred solution of 1.53g (5.0mmol) of 5-ethyl-2-phenyl-3-propargyl-6-trifluoromethyl- 4(3H)-pyrimidinone (Compound 46) in 30mL of THF was cooled to -70°C and 4.5mL of 1.6M n-BuLi in hexanes (7.2mmol) was added dropwise over 15min. The mixture was stirred at -70°C for 45min and a solution of 1.50g (6.7mmol) of N-iodosuccinimide in 10mL of THF was added dropwise over 15min. The mixture was stirred at -70°C for 45min and at room temperature for 30min. The mixture was diluted with 175mL of ether, washed with two 50mL portions of water and 50mL of saturated aqueous $NaHCO_3$ and dried over $MgSO_4$. Removal of the solvent left 2.36g of crude product as a brown solid which was purified by flash chromatography on 30g of silica gel, eluting with 0, 10, 20, 30 and 40% ether in hexanes, to

furnish 0.96g (44%) of 5-ethyl-3-(3-iodopropargyl)-2-phenyl-6-trifluoromethyl-4(3H)-pyrimidinone (Compound 219) as a an off-white solid, m.p. 120 - 125°C (dec).$^1$H-NMR ($CDCl_3$) $\delta$ 1.25(3H,t), 2.75(2H,q), 4.75 (2H,s), 7.5 - 7.7 (5H).

Methods of Use.

In another aspect, this invention relates to a method of controlling weeds comprising applying to said weed or the locus of said weed or to the surface of the growth medium of said weed a herbicidally effective amount of a compound or composition of the invention.

The compounds of the invention are useful as preemergence and postemergence herbicides. In general, they require lower doses to control weeds preemergence. Preemergence herbicides are usually applied to the soil either before, during or after seeding, but before the crop emerges. Postemergence herbicides are applied after the plants have emerged and during their growth period. The embodied materials generally show selectivity to several agronomically important crops such as corn, cotton, rice, soybean, sugarbeet, sunflower, peanut and wheat.

Under some conditions the compounds of the invention may be incorporated into the soil or other growth medium prior to planting a crop. This incorporation may be by any convenient means, including mixing with the soil, applying the compound to the surface of the soil and then dishing or dragging into the soil to the desired depth, or by employing a liquid carrier.

The 2-arylpyrimidines of the present invention can be applied to various loci such the soil or the foliage. For such purposes these compounds can be used in the technical or pure form as prepared, as solutions or as formulations. The compounds are usually taken up in a carrier or are formulated so as to render them suitable for subsequent dissemination as herbicides. For example, these chemical agents can be formulated as wettable powders, emulsifiable concentrates, dusts, granular formulations, aerosols, or flowable emulsion concentrates. In such formulations, the compounds are extended with a liquid or solid carrier and, when desired, suitable surfactants are incorporated.

It is usually desirable, particularly in the case of foliar spray formulations, to include adjuvants, such as wetting agents, spreading agents, dispersing agents, stickers, adhesive and the like in accordance with agricultural practices. Such adjuvants commonly used in the art can be found in the John W. McCutcheon, Inc. publication "Detergents and Emulsifiers, Annual", Allured Publishing Company, Ridgewood, New Jersey, U.S.A.

The 2-substitutes pyrimidines can be applied as herbicidal sprays by methods commonly employed, such as conventional high-gallonage hydraulic sprays, low-gallonage sprays, air-blast spray, aerial sprays and dusts. The dilution and rate of application will depend upon the type of equipment employed, the method of application and weeds to be controlled, but the preferred effective amount is usually from about 0.011 to about 11.2 kg/hectare (about 0.01 lb. to about 10 lbs. per acre) of the active ingredient.

As a soil treatment the chemical can be incorporated in the soil or applied to the surface usually at a rate of from about 0.011 to about 11.2 kg/hectare (about 0.01 to about 10 lbs. per acre). As a foliar spray, the toxicant is usually applied to growing plants at a rate of from about 0.011 to about 11.2 kg/hectare (about 0.01 to about 10 lbs. per acre).

The 2-substituted pyrimidines of the invention can also be mixed with fertilizers or fertilizing materials before their application. In one type of solid fertilizing composition in which the 2-substituted pyrimidines can be used, particles of a fertilizer or fertilizing ingredients, such as ammonium sulfate, ammonium nitrate, or ammonium phosphate, can be coated with one or more of the compounds. The solid compounds and solid fertilizing material can also be admixed in mixing or blending equipment, or they can be incorporated with fertilizers in granular formulations. Any relative proportion of fertilizer can be used which is suitable for the crops and weeds to be treated. The 2-substituted pyrimidine will commonly be from about 5% to about 25% of the fertilizing composition. These compositions provide fertilizing materials which promote the rapid growth of desired plants, and at the same time control the growth of undesired plants.

For some applications, one or more other herbicides may be added to the herbicides of the present invention, thereby providing additional advantages and effectiveness. When mixtures of herbicides are employed, the relative proportions which are used will depend upon the relative efficacy of compounds in the mixture with respect to the plants to be treated. Examples of other herbicides which can be combined with those of the present invention include:

**CARBOXYLIC ACIDS AND DERIVATIVES**

2,3,6-trichlorobenzoic acid and its salts;
2,3,5,6-tetrachlorobenzoic acid and its salts;
2-methoxy-3,5,6-trichlorobenzoic acid and its salts;
2-methoxy-3,6-dichlorobenzoic acid and its salts;
2-methyl-3,6-dichlorobenzoic acid and its salts;
2,3-dichloro-6-methylbenzoic acid and its salts;
2,4-dichlorophenoxyacetic acid and its salts and esters;
2,4,5-trichlorophenoxyacetic acid and its salts and esters;

2-methyl-4-chlorophenoxyacetic acid and its salts and esters;
2-(2,4,5-trichlorophenoxy)propionic acid and its salts and esters;
4-(2,4-dichlorophenoxy)butyric acid and its salts and esters;
4-(2-methyl-4-chlorophenoxy)butyric acid and its salts and esters;
2,3,6-trichlorophenylacetic acid and its salts;
3,6-endoxohexahydrophthalic acid and its salts;
dimethyl 2,3,5,6-tetrachloroterephthalate; trichloroacetic acid and its salts;
2,2-dichloropropionic acid and its salts;
2,3-dichloroisobutyric acid and its salts;
isopropylammonium 2-(4-isopropyl-4-methyl-5-oxo-2-imidazolin-2-yl)nicotinate;
2-[4,5-dihydro-4-methyl-4-(1-methylethyl)-5-oxo-1H-imidazol-2-yl]-3-quinolinecarboxylic acid;
6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester;
N-(phosphomethyl)glycine isopropylammonium salt;
[3,5,6-trichloro-(2-pyridinyl)oxy]acetic acid;
3,7-dichloro-8-quinolinecarboxylic acid;
ammonium DL-homoalanin-4-yl(methyl)phosphinate;

**CARBAMIC ACID DERIVATIVES**

ethyl N,N-di(*n*-propyl)thiolcarbamate;
*n*-propyl N,N-di(*n*-propyl)thiolcarbamate;
ethyl N-ethyl-N-(*n*-butyl)thiolcarbamate;
*n*-propyl N-ethyl-N-(*n*-butyl)thiolcarbamate;
2-chloroallyl N,N-diethyldithiocarbamate;
isopropyl N-phenylcarbamate;
isopropyl N-(*m*-chlorophenyl)carbamate;
4-chloro-2-butynyl-N-(*m*-chlorophenyl)carbamate;
methyl N-(3,4-dichlorophenyl)carbamate;
dinitro-*o*-(sec-butyl)phenol and its salts;
pentachlorophenol and its salts
S-(4-chlorobenzyl)-N,N-diethylthiolcarbamate;

**SUBSTITUTED UREAS**

2-chloro-N- [(4-methoxy-6-methyl-1,3,5-triazin-2-yl)aminocarbonyl] -benzenesulfonamide;
3-(3,4-dichlorophenyl)-1,1-dimethylurea;
3-phenyl-1,1-dimethylurea;
3-(3,4-dichlorophenyl)-3-methoxy-1,1-dimethylurea;
3-(4-chlorophenyl)-3-methoxy-1,1-dimethylurea;
3-(3,4-dichlorophenyl)-1-n-butyl-1-methylurea;
3-(3,4-dichlorophenyl)-1-methoxy-1-methylurea;
3-(4-chlorophenyl)-1-methoxy-1-methylurea;
3-(3,4-dichlorophenyl)-1,1,3-trimethylurea;
3-(3,4-dichlorophenyl)diethylurea;
N-(4-isopropylphenyl)-N,N'-dimethylurea;
dichloral urea;
methyl 2-[[[[(4,6-dimethyl-2-pyrimidinyl)amino]-carbonyl]amino]sulfonyl]benzoate;
N-((6-methoxy-4-methyl-1,3,5-triazin-2-yl)aminocarbonyl)-2-(2-chloroethoxy)-benzenesulfonamide;
2-[[[(4-chloro-6-methoxypyrimidine-2-yl)aminocarbonyl]amino]-sulfonyl]benzoic acid, ethyl ester;
methyl 2-[[[[(4-methoxy-6-methyl-1,3,5-triazin-2-yl)amino]-carbonyl]amino]-sulfonyl]benzoate;
methyl 3-[[(4-methoxy-6-methyl-1,3,5-triazin-2-yl)aminocarbonyl]aminosulfonyl]-2-thio-phenecarboxylate;
methyl 2-[[[[[(4,6-dimethoxypyrimidin-2-yl)aminolcarbonyl]aminojsulfonyl]-methyl]benzoate;
methyl 2-[[[[(4-methoxy-6-methyl-1,3,5-triazin-2-yl)methylamino]carbonyl]amino]-sulfonyl]benzoate;

**SUBSTITUTED TRIAZINES**

2-chloro-4,6-bis(ethylamino)-s-triazine;

2-chloro-4-ethylamino-6-isopropylamino-s-triazine;
2-chloro-4,6-bis(3-methoxy-n-propylamino)-s-triazine;
2-methoxy-4,6-bis(isopropylamino)-s-triazine;
2-chloro-4-ethylamino-6-(3-methoxy-*n*-propylamino)-s-triazine;
2-methylmercapto-4,6-bis(isopropylamino)-s-triazine;
2-methylmercapto-4,6-bis(ethylamino)-2-triazine;
2-methylmercapto-4-ethylamino-6-isopropylamino-s-triazine;
2-chloro-4,6-bis(isopropylamino)-s-triazine;
2-methoxy-4-ethylamino-6-isopropylamino-s-triazine;
2-methylmercapto-4-(2-methoxyethylamino)-6-isopropylamino-s-triazine;
4-amino-6-(t-butyl)-3-(methylthio)-1,2,4-triazine-5(4H)-one;

**DIPHENYL ETHER DERIVATIVES**

2,4-dichloro-4'-nitrodiphenyl ether;
2,4,6-trichloro-4'-nitrodiphenyl ether;
2,4-dichloro-6-fluoro-4'-nitrodiphenyl ether;
3-methyl-4'-nitrodiphenyl ether;
3,5-dimethyl-5'-nitrodiphenyl ether;
2,4'-dinitro-4-(trifluoromethyl)diphenyl ether;
2,4-dichloro-3'-methoxy-4'-nitrodiphenyl ether;
sodium 5-(2-chloro-4-(trifluoromethyl)phenoxy)-2-nitrobenzoate;
2-chloro-1-(3-ethoxy-4-nitrophenoxy)-4-(trifluoromethyl)benzene;
1-(carboethoxy)ethyl 5-[2-chloro-4-(trifluoromethyl)-phenoxy]-2-nitrobenzoate;
5-[2-chloro-4-(trifluoromethyl)phenoxyl]-N-(methylsulphonyl)-2-nitrobenzamide;

**ANILIDES**

2-chloro-N-(2-ethyl-6-methylphenyl)-N-(2-methoxy-1-methylethyl)acetamide;
2-chloro-2',6'-diethyl-N-(2-propyloxyethyl)acetanilide;
N-(3,4-dichlorophenyl)propionamide;
N-(3,4-dichlorophenyl)methacrylamide;
N-(3-chloro-4-methylphenyl)-2-methylpentanamide;
N-(3,4-dichlorophenyl)trimethylacetamide;
N-(3,4-dichlorophenyl)-alpha,alpha-dimethylvaleramide;
N-isopropyl-N-phenylchloroacetamide;
N-n-butoxymethyl-N-(2,6-diethylphenyl)chloroacetamide;
N-methoxymethyl-N-(2,6-diethylphenyl)chloroacetamide;

**OXYPHENOXY HERBICIDES**

2-(4-(2,4-dichlorophenoxy)phenoxy)methyl propionate;
methyl 2-(4-(3-chloro-5-(trifluoromethyl)-2-pyridinyloxy)phenoxy)propanoate;
butyl (R)-2-[4-[5-(trifluoromethyl)-2-pyridinyloxy]-phenoxy]propionate;
ethyl 2-[4-[(6-chloro-2-benzoxazolyl)oxy]phenoxy]propanoate;
butyl 2-[4-[[5-(trifluoromethyl)-2-pyridinyl]oxy]phenoxy]propionate;
2-[4-[(6-chloro-2-quinoxalinyl)oxy]phenoxy]propionicacid, ethyl ester;

**URACILS**

5-bromo-3-s-butyl-6-methyluracil;
5-bromo-3-cyclohexyl-1,6-dimethyluracil;
3-cyclohexyl-5,6-trimethyleneuracil;
5-bromo-3-isopropyl-6-methyluracil;
3-tert-butyl-5-chloro-6-methyluracil;

**NITRILES**

2,6-dichlorobenzonitrile;
diphenylacetonitrile;
3,5-dibromo-4-hydroxybenzonitrile;
3,5-diiodo-4-hydroxybenzonitrile;

**OTHER ORGANIC HERBICIDES**

2-chloro-N,N-diallylacetamide;
N-(1,1-dimethyl-2-propynyl)-3,5-dichlorobenzamide;
maleic hydrazide;
3-amino-1,2,4-triazole; monosodium methanearsonate;
disodium methanearsonate;
N,N-dimethyl-alpha,alpha-diphenylacetamide;
N-N-di(n-propyl)-2,6-dinitro-4-(trifluoromethyl)aniline;
N,N-di(n-propyl)-2,6-dinitro-4-methylaniline;
N,N-di(n-propyl)-2,6-dinitro-4-methylsulfonylaniline;
O-(2,4-dichlorophenyl)-O-methyl isopropylphosphoramidothioate;
4-amino-3,5,6-trichloropicolinic acid;
2,3-dichloro-1,4-naphthoquinone;
di(methoxythiocarbonyl)disulfide;
3-(1-methylethyl)-1H-2,1,3-benzothiadiazin-(4)3H-one-2,2-dioxide;
6,7-dihydrodipyridol[1,2-a:2',1'-c]pyrazidiium salts;
1,1'-dimethyl-4,4'-bipyridinium salts;
3,4,5,6-tetrahydro-3,5-dimethyl-2-thio-2H-1,3,5-thiadiazine;
2-[1-(ethoxyimino)butyl]-5-[s-(ethylthio)propyl]-3-hydroxy-2-cyclohexen-1-one;
2-(2-chlorophenyl)methyl-4,4-dimethyl-3-isoxazolidinone;
N-(1-ethylpropyl)-3,4-dimethyl-2,6-dinitrobenzamide;
4-chloro-5-(methylamino)-2-(a,a,a-trifluoro-*m*-toluyl)-3-(2H)-pyridazinone;
2-(3,5-dichlorophenyl)-2-(2,2,2-trichloromethyl)oxirane.

When mixtures of herbicides are employed, the relative proportions which are used will depend upon the crop to be treated and the degree of selectivity in weed control desired. The herbicidal activity of the 2-substituted pyrimidines of the present invention towards a number of common weeds was evaluated using a greenhouse method of testing. Using the procedures described below, the aryl pyrimidines of the present invention were evaluated for control of weeds selected from the following:

**Monocots**

| | |
|---|---|
| Barnyardgrass (BYG) | *Echinochloa crus-galli* |
| Crabgrass (CRB) | *Digitaria sanguinilis* |

| | |
|---|---|
| Foxtail (FOX) | Setaria viridis |
| Johnsongrass (JON) | Sorghum halepense |
| Meadow Foxtail (MF) | Alopecurus pratensis |
| Nutsedge (NUT) | Cyperus esculentus |
| Wild Oat (WO) | Avena fatua |

Dicots

| | |
|---|---|
| Beggartick (BID) | Bidens pilosa |
| Cocklebur (CKL) | Xanthium strumarium |
| Morningglory (MG) | Ipomoea lacunosa |
| Nightshade (NS) | Solanum nigrum |
| Pigweed (PIG) | Amaranthus retroflexus |
| Smartweed (SMT) | Polygonum lapathifolium |
| Velvetleaf (VEL) | Abutilon theophrasti |

The following test procedure was employed. Seeds of selected plants were planted in flats or pots. For preemergence tests, immediately after planting, the test compound was sprayed directly onto the soil surface. The flats or pots were placed in the greenhouse and then watered. For postemergence tests, the seeds were allowed to germinate and grow for 10 to 21 days. Before application, each series of test plants were selected for uniformity, size and stage of development. The test plants were then treated with the test compound, returned to the greenhouse and watered.

The compound to be evaluated was dissolved in an appropriate solvent, usually acetone, and sprayed over the flats or pots using a carrier volume equivalent to 233.84 or 467.68 litres/hectare (25 or 50 gallons per acre) at the rate of application in pounds per acre (Lb/A) or grams per hectare (g/Ha) specified in the below tables. About two or three weeks after application of the test compound, the state of growth of the plant was observed. Each species was evaluated on a scale of 0-100 in which 0 equals no activity and 100 equals total control. The column heading abbreviations in the below tables for the plants tested are the same as for the monocots and dicots hereinabove. The dash ("-") entry signifies no testing for the specified conditions.

TABLE 3

| EX. | TYPE | lb/a* | CKL | MG | PIG | SMT | VEL | BYG | FOX | JON | NUT | WO |
|---|---|---|---|---|---|---|---|---|---|---|---|---|
| 1 | PRE | 4.00 | 20 | 100 | 100 | 100 | 100 | 100 | 100 | 99 | 55 | 100 |
| | POST | 4.00 | 25 | 70 | 100 | 100 | 55 | 80 | 50 | 0 | 15 | 100 |
| 2 | PRE | 4.00 | 5 | 100 | 100 | 100 | 100 | 100 | 100 | 100 | 65 | 60 |
| | POST | 4.00 | 15 | 75 | 75 | 50 | 35 | 80 | 75 | 0 | 0 | 35 |
| 4 | PRE | 4.00 | 25 | 100 | 100 | 100 | 100 | 100 | 100 | 100 | 85 | 100 |
| | POST | 4.00 | 20 | 45 | 100 | 55 | 50 | 95 | 95 | 70 | 35 | 90 |
| 5 | PRE | 4.00 | 40 | 100 | 100 | 100 | 100 | 100 | 100 | 100 | 100 | 98 |
| | POST | 4.00 | 20 | 85 | 70 | 99 | 65 | 98 | 95 | 65 | 80 | 70 |
| 6 | PRE | 4.00 | 15 | 100 | 100 | 100 | 100 | 100 | 100 | 100 | 100 | 85 |

* 4 lb/a = 4.484 × $10^3$g/hectare

TABLE 3 (continued)

| EX. | TYPE | lb/a* | CKL | MG | PIG | SMT | VEL | BYG | FOX | JON | NUT | WO |
|---|---|---|---|---|---|---|---|---|---|---|---|---|
| | POST | 4.00 | 0 | 10 | 45 | 40 | 0 | 20 | 0 | 70 | 15 | 0 |
| 7 | PRE | 4.00 | 0 | 10 | _ | 100 | 60 | 90 | 100 | 55 | _ | 40 |
| | POST | 4.00 | 0 | 0 | 20 | 15 | 20 | 0 | 0 | 0 | 0 | 0 |

"_" EQUALS NOT TESTED

* 4 lb/a = 4.484 × $10^3$g/hectare

# TABLE 3A

| EX. | TYPE | lb/a** | BID | NS | SMT | VEL | BYG | CRB | FOX | MF |
|---|---|---|---|---|---|---|---|---|---|---|
| 8 | PRE | 4.00 | 100 | 100 | 100 | 100 | 100 | 100 | 100 | - |
| | POST | 4.00 | 15 | 100 | 5 | 5 | 20 | 20 | 75 | 65 |

| EX. | TYPE | lb/a** | BID | NS | SMT | VEL | BYG | CRB | FOX | MF |
|---|---|---|---|---|---|---|---|---|---|---|
| 9 | PRE | 2.00 | - | 80 | 100 | 10 | 100 | - | 100 | - |
| | POST | 2.00 | - | 30 | 0 | 0 | 0 | - | 0 | - |
| 11 | PRE | 2.00 | - | 100 | 100 | 100 | 100 | - | 100 | - |
| | POST | 2.00 | - | 45 | 0 | 0 | 15 | - | 15 | - |
| 12 | PRE | 1.00 | 25 | 100 | 100 | 100 | 100 | 100 | 100 | 100 |
| | POST | 1.00 | 25 | 100 | 100 | 35 | 95 | 95 | 95 | 95 |
| 13 | PRE | 1.00 | 0 | 100 | 95 | 0 | 90 | 100 | 100 | 90 |
| | POST | 1.00 | 0 | 20 | 20 | 20 | 0 | 20 | 0 | 0 |
| 14 | PRE | 1.00 | 60 | 100 | 100 | 95 | 100 | 100 | 100 | 100 |
| | POST | 1.00 | 75 | 95 | 95 | 80 | 100 | 95 | 80 | 50 |
| 16 | PRE | 1.00 | 80 | 100 | 100 | 90 | 100 | 100 | 100 | 100 |
| | POST | 1.00 | 35 | 100 | 95 | 10 | 90 | 95 | 85 | 90 |
| 17 | PRE | 1.00 | 0 | 90 | 100 | 80 | 40 | 90 | 25 | 0 |
| | POST | 1.00 | 20 | 100 | 20 | 0 | 0 | 20 | 0 | 0 |
| 18 | PRE | 1.00 | 0 | 0 | 0 | 0 | 0 | 40 | 0 | 0 |
| | POST | 1.00 | 0 | 100 | 0 | 0 | 0 | 0 | 0 | 0 |
| 19 | PRE | 1.00 | 0 | 25 | 25 | 0 | 0 | 0 | 0 | 0 |
| | POST | 1.00 | 10 | 100 | 0 | 20 | 0 | 0 | 0 | 0 |

| EX. | TYPE | lb/a** | BID | NS | SMT | VEL | BYG | CRB | FOX | MF |
|---|---|---|---|---|---|---|---|---|---|---|
| 21 | PRE | 1.00 | 0 | 10 | 90 | 0 | 90 | 100 | 50 | 90 |
| | POST | 1.00 | 0 | - | 0 | 0 | 0 | 0 | 0 | 0 |
| 22 | PRE | 1.00 | 90 | 100 | 100 | 100 | 100 | 100 | 100 | 100 |
| | POST | 1.00 | 70 | - | 100 | 0 | 100 | 100 | 80 | 100 |
| 23 | PRE | 1.00 | 25 | 95 | 0 | 100 | 25 | 0 | 95 | 100 |
| | POST | 1.00 | 10 | - | 10 | 0 | 0 | 20 | 0 | 0 |
| 25 | PRE | 1.00 | 0 | - | 0 | 0 | 0 | 0 | 0 | 0 |
| | POST | 1.00 | 25 | 50 | 25 | 0 | 0 | 0 | 0 | 0 |
| 26 | PRE | 1.00 | 0 | - | 0 | 0 | 50 | 75 | 40 | 0 |
| | POST | 1.00 | 0 | 0 | 0 | 0 | 0 | 0 | 0 | 0 |
| 27 | PRE | 1.00 | 0 | - | - | 20 | 95 | 100 | 100 | 100 |
| | POST | 1.00 | 0 | 85 | 60 | 0 | 75 | 75 | 10 | 75 |
| 28 | PRE | 1.00 | 0 | - | - | 20 | 100 | 100 | 100 | 100 |
| | POST | 1.00 | 20 | 95 | 50 | 60 | 95 | 95 | 50 | 90 |
| 29 | PRE | 1.00 | 25 | - | - | 60 | 100 | 100 | 100 | 100 |
| | POST | 1.00 | 25 | 100 | 100 | 50 | 95 | 95 | 90 | 100 |
| 30 | PRE | 1.00 | 0 | 0 | 25 | 0 | 60 | 100 | 90 | 80 |
| | POST | 1.00 | 0 | 75 | 0 | 0 | 0 | 0 | 0 | 0 |

| EX. | TYPE | lb/a** | BID | NS | SMT | VEL | BYG | CRB | FOX | MF |
|---|---|---|---|---|---|---|---|---|---|---|
| 31 | PRE | 1.00 | 10 | 100 | 100 | 20 | 100 | 100 | 100 | 100 |
| | POST | 1.00 | 25 | 90 | 95 | 20 | 95 | 100 | 70 | 95 |
| 32 | PRE | 1.00 | 0 | 95 | 100 | 25 | 95 | 100 | 100 | 95 |
| | POST | 1.00 | 25 | 90 | 90 | 20 | 95 | 95 | 80 | 90 |
| 33 | PRE | 1.00 | 20 | 100 | 100 | 0 | 95 | 100 | 100 | 100 |
| | POST | 1.00 | 20 | 80 | 40 | 0 | 25 | 25 | 25 | 50 |
| 35 | PRE | 1.00 | 20 | 0 | 100 | 40 | 100 | 100 | 100 | 100 |
| | POST | 1.00 | 10 | 95 | 95 | 10 | 95 | 95 | 70 | 60 |
| 36 | PRE | 1.00 | 0 | 100 | 60 | 0 | 75 | 95 | 75 | 75 |
| | POST | 1.00 | 40 | 80 | 40 | 0 | 50 | 40 | 40 | 10 |
| 37 | PRE | 1.00 | 10 | 95 | 95 | 10 | 90 | 95 | 90 | 90 |
| | POST | 1.00 | 30 | 25 | 40 | 0 | 20 | 0 | 0 | 0 |
| 38 | PRE | 1.00 | 0 | 25 | 0 | 80 | 0 | 0 | 0 | 0 |
| | POST | 1.00 | 20 | 20 | 0 | 0 | 0 | 0 | 0 | 0 |
| 39 | PRE | 1.00 | 80 | 95 | - | 80 | 95 | 100 | 100 | 100 |
| | POST | 1.00 | 70 | 95 | 90 | 25 | 90 | 95 | 60 | 95 |
| 40 | PRE | 1.00 | 0 | 90 | - | 0 | 50 | 75 | 80 | 0 |
| | POST | 1.00 | 40 | 0 | 0 | 0 | 25 | 0 | 0 | 0 |

| EX. | TYPE | lb/a** | BID | NS | SMT | VEL | BYG | CRB | FOX | MF |
|---|---|---|---|---|---|---|---|---|---|---|
| 45 | PRE | 1.00 | 0 | 95 | 100 | 0 | 85 | 95 | 100 | 95 |
| | POST | 1.00 | 70 | 90 | 90 | 60 | 85 | 80 | 20 | 85 |
| 46 | PRE | 1.00 | 80 | 100 | 100 | 100 | 100 | 100 | 100 | 100 |
| | POST | 1.00 | 95 | 100 | 100 | 85 | 95 | 100 | 95 | 95 |
| 47 | PRE | 1.00 | 0 | 90 | 90 | 20 | 95 | 95 | 95 | 95 |
| | POST | 1.00 | 0 | 100 | 0 | 0 | 25 | 25 | 0 | 0 |
| 48 | PRE | 1.00 | 95 | 100 | 100 | 80 | 100 | 100 | 100 | 100 |
| | POST | 1.00 | 70 | 100 | 100 | 70 | 100 | 95 | 90 | 95 |
| 51 | PRE | 1.00 | 80 | 95 | 20 | 50 | 100 | 95 | 100 | 100 |
| | POST | 1.00 | 20 | 90 | 25 | 0 | 40 | 50 | 20 | 0 |
| 52 | PRE | 2.00 | - | 95 | 100 | 0 | 35 | - | 100 | - |
| | POST | 2.00 | - | 0 | 0 | 0 | 0 | - | 0 | - |
| 53 | PRE | 1.00 | 0 | 90 | - | 25 | 90 | 95 | 95 | 40 |
| | POST | 1.00 | 0 | 90 | 80 | 10 | 50 | 40 | 25 | 10 |
| 54 | PRE | 1.00 | 100 | 100 | 95 | 100 | 100 | 100 | 100 | 100 |
| | POST | 1.00 | 95 | 100 | 100 | 90 | 95 | 95 | 95 | 95 |
| 55 | PRE | 1.00 | 25 | 95 | 95 | 50 | 100 | 100 | 100 | 100 |
| | POST | 1.00 | 20 | 90 | 90 | 75 | 80 | 90 | 80 | 80 |
| 56 | PRE | 1.00 | 95 | 100 | 95 | 40 | 100 | 100 | 100 | 100 |
| | POST | 1.00 | 70 | 100 | 90 | 50 | 95 | 95 | 95 | 95 |

| EX. | TYPE | lb/a** | BID | NS | SMT | VEL | BYG | CRB | FOX | MF |
|---|---|---|---|---|---|---|---|---|---|---|
| 57 | PRE | 1.00 | 25 | 100 | 85 | 25 | 100 | 100 | 100 | 100 |
| | POST | 1.00 | 25 | 100 | 90 | 50 | 90 | 95 | 80 | 90 |
| 58 | PRE | 1.00 | 95 | 100 | 95 | 100 | 100 | 100 | 100 | 100 |
| | POST | 1.00 | 80 | 80 | 90 | 85 | 80 | 100 | 75 | 70 |
| 59 | PRE | 1.00 | 20 | 100 | 95 | 80 | 100 | 100 | 100 | 100 |
| | POST | 1.00 | 20 | 70 | 40 | 20 | 80 | 90 | 40 | 70 |
| 60 | PRE | 1.00 | 40 | 100 | 85 | 40 | 95 | 100 | 100 | 100 |
| | POST | 1.00 | 25 | 95 | 85 | 25 | 70 | 95 | 60 | 40 |
| 61 | PRE | 1.00 | 0 | 70 | 0 | 0 | 0 | 0 | 0 | 0 |
| | POST | 1.00 | 0 | 80 | 20 | 0 | 20 | 0 | 20 | 0 |
| 62 | PRE | 1.00 | 90 | 100 | 90 | 100 | 100 | 100 | 100 | 100 |
| | POST | 1.00 | 80 | 100 | 100 | 90 | 90 | 95 | 95 | 100 |
| 63 | PRE | 1.00 | 0 | 90 | 10 | 0 | 0 | 10 | 20 | 0 |
| | POST | 1.00 | 10 | 10 | 0 | 0 | 0 | 20 | 0 | 0 |
| 64 | PRE | 1.00 | 20 | 100 | 95 | 50 | 100 | 100 | 100 | 100 |
| | POST | 1.00 | 60 | 100 | 100 | 75 | 95 | 95 | 95 | 95 |
| 65 | PRE | 1.00 | 10 | 100 | 100 | 20 | 100 | 100 | 100 | 100 |
| | POST | 1.00 | 40 | 100 | 75 | 50 | 95 | 95 | 95 | 90 |
| 66 | PRE | 1.00 | 10 | 95 | 100 | 100 | 100 | 100 | 100 | 100 |
| | POST | 1.00 | 70 | 100 | 100 | 80 | 95 | 100 | 90 | 95 |

| EX. | TYPE | lb/a** | BID | NS | SMT | VEL | BYG | CRB | FOX | MF |
|---|---|---|---|---|---|---|---|---|---|---|
| 67 | PRE | 1.00 | 0 | 95 | 80 | 10 | 95 | 100 | 100 | 100 |
| | POST | 1.00 | 10 | 100 | 80 | 20 | 90 | 95 | 90 | 90 |
| 68 | PRE | 1.00 | 50 | 100 | 100 | 40 | 100 | 100 | 100 | 100 |
| | POST | 1.00 | 70 | 100 | 85 | 70 | 95 | 95 | 85 | 85 |
| 69 | PRE | 1.00 | - | - | 100 | 0 | 0 | 0 | 0 | 0 |
| | POST | 1.00 | 0 | 50 | 10 | 0 | 0 | 0 | 0 | 0 |
| 70 | PRE | 1.00 | 0 | 100 | 100 | 50 | 100 | 100 | 100 | 100 |
| | POST | 1.00 | 25 | 80 | 85 | 60 | 70 | 90 | 60 | 50 |
| 71 | PRE | 1.00 | 20 | 100 | 100 | 0 | 100 | 100 | 100 | 100 |
| | POST | 1.00 | 40 | 95 | 70 | 60 | 80 | 95 | 60 | 70 |
| 76 | PRE | 1.00 | 0 | 100 | 90 | 85 | 95 | 100 | 100 | 100 |
| | POST | 1.00 | 25 | 100 | 95 | 25 | 85 | 75 | 70 | 75 |
| 77 | PRE | 1.00 | 20 | 100 | 90 | 100 | 100 | 100 | 100 | 100 |
| | POST | 1.00 | 90 | 100 | 100 | 85 | 95 | 90 | 90 | 90 |
| 80 | PRE | 1.00 | 0 | 90 | 20 | 0 | 60 | 95 | 95 | 70 |
| | POST | 1.00 | 10 | 95 | 20 | 40 | 0 | 0 | 20 | 0 |
| 81 | PRE | 1.00 | 0 | 0 | 0 | 0 | 0 | 90 | 0 | 0 |
| | POST | 1.00 | 20 | 20 | 0 | 0 | 0 | 0 | 0 | 0 |

| EX. | TYPE | lb/a** | BID | NS | SMT | VEL | BYG | CRB | FOX | MF |
|---|---|---|---|---|---|---|---|---|---|---|
| 83 | PRE | 1.00 | 0 | 90 | 90 | 0 | 70 | 95 | 40 | 30 |
| | POST | 1.00 | 30 | 50 | 20 | 10 | 0 | 0 | 0 | 0 |
| 84 | PRE | 1.00 | 75 | 95 | 60 | 80 | 90 | 100 | 100 | 100 |
| | POST | 1.00 | 50 | 100 | 90 | 50 | 85 | 90 | 75 | 75 |
| 85 | PRE | 1.00 | 60 | 80 | 95 | 70 | 100 | 100 | 100 | 100 |
| | POST | 1.00 | 90 | 100 | 95 | 75 | 95 | 100 | 90 | 90 |
| 87 | PRE | 1.00 | 0 | 0 | 0 | 0 | 80 | 90 | 100 | 90 |
| | POST | 1.00 | 0 | 20 | 0 | 0 | 25 | 95 | 0 | 0 |
| 88 | PRE | 1.00 | 0 | 90 | 85 | 60 | 95 | 100 | 100 | 95 |
| | POST | 1.00 | 40 | 100 | 70 | 80 | 85 | 95 | 75 | 80 |
| 89 | PRE | 1.00 | 0 | 0 | 0 | 0 | 0 | 100 | 0 | 0 |
| | POST | 1.00 | 0 | 70 | 0 | 0 | 0 | 0 | 0 | 0 |
| 90 | PRE | 1.00 | 80 | 100 | 100 | 20 | 100 | 100 | 100 | 100 |
| | POST | 1.00 | 80 | 100 | 90 | 35 | 95 | 95 | 90 | 90 |
| 91 | PRE | 1.00 | 0 | 0 | 0 | 0 | 10 | 80 | 10 | 0 |
| | POST | 1.00 | 0 | 0 | 0 | 0 | 0 | 0 | 0 | 0 |
| 92 | PRE | 1.00 | 0 | 0 | 0 | 0 | 0 | 100 | 0 | 0 |
| | POST | 1.00 | 0 | 0 | 0 | 0 | 0 | 0 | 0 | 0 |
| 94 | PRE | 1.00 | 0 | 100 | 100 | 70 | 100 | - | 100 | 100 |
| | POST | 1.00 | 70 | 100 | 90 | 60 | 90 | 90 | 85 | 80 |

| EX. | TYPE | lb/a** | BID | NS | SMT | VEL | BYG | CRB | FOX | MF |
|---|---|---|---|---|---|---|---|---|---|---|
| 95 | PRE | 1.00 | 60 | 90 | 95 | 70 | 100 | - | 85 | 100 |
| | POST | 1.00 | 70 | 100 | 85 | 80 | 95 | 90 | 85 | 90 |
| 96 | PRE | 1.00 | 20 | 100 | - | 40 | 90 | 95 | 90 | 95 |
| | POST | 1.00 | 60 | 100 | 90 | 75 | 95 | 95 | 50 | 90 |
| 97 | PRE | 1.00 | 60 | 100 | - | 25 | 100 | 100 | 100 | 100 |
| | POST | 1.00 | 85 | 100 | - | 100 | 100 | 100 | 100 | 100 |
| 98 | PRE | 1.00 | 80 | 100 | - | 100 | 100 | 100 | 100 | 100 |
| | POST | 1.00 | 90 | 100 | 90 | 85 | 95 | 95 | 90 | 90 |
| 99 | PRE | 1.00 | 0 | 100 | 80 | 10 | 100 | 95 | 100 | 100 |
| | POST | 1.00 | 0 | 100 | 90 | 0 | 80 | 95 | 20 | 90 |
| 103 | PRE | 1.00 | 10 | 95 | 95 | 75 | 100 | 100 | 100 | 100 |
| | POST | 1.00 | 40 | 95 | 100 | 25 | 100 | 95 | 85 | 95 |
| 105 | PRE | 1.00 | 0 | 0 | 0 | 0 | 0 | 0 | 0 | 0 |
| | POST | 1.00 | 0 | 25 | 0 | 0 | 0 | 25 | 0 | 0 |
| 106 | PRE | 1.00 | 25 | 90 | 50 | 100 | 100 | - | 100 | 100 |
| | POST | 1.00 | 50 | 80 | 70 | 60 | 85 | 90 | 70 | 60 |
| 107 | PRE | 1.00 | 95 | 95 | 70 | 50 | 90 | - | 95 | 95 |
| | POST | 1.00 | 40 | 80 | 85 | 75 | 90 | 85 | 35 | 60 |
| 108 | PRE | 1.00 | 95 | 100 | 95 | 70 | 100 | 100 | 100 | 100 |
| | POST | 1.00 | 85 | 85 | 80 | 40 | 65 | 90 | 50 | 50 |

| EX. | TYPE | lb/a** | BID | NS | SMT | VEL | BYG | CRB | FOX | MF |
|---|---|---|---|---|---|---|---|---|---|---|
| 110 | PRE | 1.00 | 100 | 100 | 100 | 100 | 100 | 100 | 100 | 100 |
| | POST | 1.00 | 75 | 100 | 85 | 70 | 90 | 95 | 85 | 80 |
| 111 | PRE | 1.00 | 100 | 40 | 20 | 0 | 20 | 100 | 50 | 0 |
| | POST | 1.00 | 0 | 60 | 0 | 0 | 10 | 0 | 0 | 0 |
| 112 | PRE | 1.00 | 0 | 100 | 0 | 0 | 80 | - | 95 | - |
| | POST | 1.00 | 0 | 75 | 60 | 35 | 25 | 75 | 15 | 10 |
| 113 | PRE | 1.00 | 40 | 100 | 40 | 0 | 20 | 0 | 0 | 0 |
| | POST | 1.00 | 0 | 80 | 50 | 20 | 35 | 20 | 0 | 10 |
| 114 | PRE | 1.00 | 0 | 80 | 20 | 0 | 50 | 95 | 60 | 50 |
| | POST | 1.00 | 0 | 80 | 40 | 20 | 40 | 30 | 0 | 0 |
| 116 | PRE | 1.00 | 50 | 100 | 100 | 60 | 100 | 100 | 100 | 100 |
| | POST | 1.00 | 25 | 95 | 100 | 85 | 90 | 95 | 80 | 95 |
| 117 | PRE | 1.00 | 80 | 100 | 40 | 35 | 95 | 95 | 100 | 95 |
| | POST | 1.00 | 20 | 90 | 25 | 35 | 50 | 60 | 30 | 25 |
| 118 | PRE | 1.00 | 50 | 95 | 80 | 0 | 95 | 100 | 95 | 95 |
| | POST | 1.00 | 60 | 90 | 95 | 40 | 90 | 95 | 85 | 90 |
| 119 | PRE | 1.00 | 85 | 95 | 80 | 0 | 90 | 100 | 100 | 95 |
| | POST | 1.00 | 80 | 90 | 95 | 70 | 90 | 95 | 70 | 75 |

| EX. | TYPE | lb/a** | BID | NS | SMT | VEL | BYG | CRB | FOX | MF |
|---|---|---|---|---|---|---|---|---|---|---|
| 120 | PRE | 1.00 | 0 | 0 | 0 | 0 | 25 | 20 | 20 | 0 |
| | POST | 1.00 | 25 | 80 | 60 | 50 | 40 | 40 | 25 | 10 |
| 121 | PRE | 1.00 | 90 | 95 | 40 | 25 | 85 | 95 | 100 | 80 |
| | POST | 1.00 | 25 | 100 | 100 | 75 | 90 | 90 | 80 | 85 |
| 122 | PRE | 1.00 | 80 | 95 | 70 | 60 | 100 | 100 | 100 | 100 |
| | POST | 1.00 | 50 | 90 | 80 | 60 | 85 | 85 | 80 | 60 |
| 123 | PRE | 1.00 | 100 | 100 | 95 | 40 | 100 | 100 | 100 | 100 |
| | POST | 1.00 | 75 | 100 | 100 | 35 | 95 | 90 | 85 | 85 |
| 125 | PRE | 1.00 | 95 | 100 | 95 | 40 | 100 | 100 | 100 | 100 |
| | POST | 1.00 | 85 | 100 | 100 | 80 | 95 | 95 | 90 | 90 |
| 126 | PRE | 1.00 | 95 | 95 | 50 | 0 | 100 | 100 | 100 | 100 |
| | POST | 1.00 | 30 | 90 | 80 | 70 | 95 | 95 | 95 | 95 |
| 128 | PRE | 1.00 | 0 | 85 | 20 | 0 | 60 | 85 | 95 | 85 |
| | POST | 1.00 | 80 | 80 | 60 | 40 | 10 | 70 | 25 | 10 |
| 129 | PRE | 1.00 | 100 | 100 | 60 | 0 | 100 | 95 | 100 | 100 |
| | POST | 1.00 | 40 | 95 | 85 | 75 | 95 | 90 | 85 | 85 |

| EX. | TYPE | lb/a** | BID | NS | SMT | VEL | BYG | CRB | FOX | MF |
|---|---|---|---|---|---|---|---|---|---|---|
| 130 | PRE | 1.00 | 95 | 100 | 100 | 60 | 100 | 100 | 100 | 100 |
| | POST | 1.00 | 35 | 100 | 100 | 95 | 95 | 95 | 90 | 95 |
| 131 | PRE | 1.00 | 0 | 20 | 0 | 0 | 80 | 95 | 60 | 50 |
| | POST | 1.00 | 0 | 80 | 25 | 25 | 20 | 70 | 0 | 0 |
| 132 | PRE | 1.00 | 0 | 100 | 95 | 10 | 100 | 100 | 100 | 100 |
| | POST | 1.00 | 40 | 100 | 100 | 80 | 90 | 95 | 85 | 95 |

"-" MEANS NOT TESTED

* 1 lb/a = $1.121 \times 10^3$ g/hectare ; 2 lb/a = $2.242 \times 10^3$ g/hectare ; and 4 lb/a = $4.484 \times 10^3$ g/hectare.

# TABLE 3B

| EXAMPLE | TYPE | g/HA | BID | BYG | CRB | FOX | MF | NS | SMT | VEL |
|---|---|---|---|---|---|---|---|---|---|---|
| 133 | POST | 1200 | 40 | 20 | 70 | 0 | 0 | 80 | 50 | 40 |
| | PRE | 1200 | 20 | 60 | 100 | 40 | 10 | 100 | 20 | 0 |
| 134 | POST | 1200 | 80 | 85 | 95 | 85 | 80 | 90 | 85 | 80 |
| | PRE | 1200 | 100 | 100 | 100 | 100 | 100 | 100 | 100 | 100 |
| 135 | POST | 1200 | 25 | 90 | 100 | 70 | 75 | 90 | 75 | 40 |
| | PRE | 1200 | 95 | 100 | 100 | 100 | 100 | 100 | 100 | 0 |
| 136 | POST | 1200 | 85 | 90 | 100 | 95 | 95 | 95 | 85 | 60 |
| | PRE | 1200 | 95 | 95 | 95 | 95 | 95 | 100 | 95 | 25 |
| 137 | POST | 1200 | 60 | 0 | 85 | 0 | 0 | 80 | 70 | 10 |
| | PRE | 1200 | 25 | 90 | 95 | 95 | 95 | 100 | 75 | 0 |
| 138 | POST | 1200 | 0 | 0 | 0 | 0 | 0 | 25 | 0 | 0 |
| | PRE | 1200 | 70 | 0 | 0 | 0 | 0 | 25 | 0 | 0 |
| 139 | POST | 1200 | 0 | 0 | 0 | 0 | 0 | 0 | 0 | 0 |
| | PRE | 1200 | 0 | 10 | 75 | 0 | 0 | 85 | 0 | 0 |
| 140 | POST | 1200 | 25 | 90 | 100 | 100 | 95 | 100 | 100 | 80 |
| | PRE | 1200 | 0 | 100 | 100 | 100 | 100 | 100 | 100 | 80 |
| 141 | POST | 1200 | 0 | 0 | 0 | 0 | 0 | 70 | 10 | 0 |
| | PRE | 1200 | 100 | 0 | 100 | 100 | 25 | 100 | 95 | 0 |
| 142 | POST | 1200 | 0 | 0 | 0 | 0 | 0 | 0 | 0 | 0 |
| | PRE | 1200 | 0 | 0 | 60 | 95 | 0 | 0 | 0 | 0 |
| 143 | POST | 1200 | 25 | 85 | 90 | 90 | 90 | 95 | 85 | 75 |
| | PRE | 1200 | 100 | 100 | 100 | 100 | 100 | 100 | 100 | 100 |

| COMPOUND | TYPE | g/HA | BID | BYG | CRB | FOX | MF | NS | SMT | VEL |
|---|---|---|---|---|---|---|---|---|---|---|
| 144 | POST | 1200 | 20 | 85 | 95 | 40 | 80 | 100 | 90 | 10 |
| | PRE | 1200 | 25 | 100 | 100 | 100 | 100 | 100 | 40 | 0 |
| 145 | POST | 1200 | 50 | 100 | 100 | 95 | 95 | 100 | 50 | 40 |
| | PRE | 1200 | 100 | 100 | 100 | 100 | 100 | 100 | 100 | 50 |
| 146 | POST | 1200 | 25 | 95 | 100 | 90 | 95 | 100 | 75 | 20 |
| | PRE | 1200 | 100 | 100 | 100 | 100 | 100 | 100 | 100 | 80 |
| 147 | POST | 1200 | 10 | 100 | 95 | 80 | 95 | 100 | 100 | 40 |
| | PRE | 1200 | 95 | 100 | 100 | 100 | 100 | 100 | 100 | 95 |
| 148 | POST | 1200 | 25 | 90 | 90 | 95 | 95 | 100 | 100 | 80 |
| | PRE | 1200 | 100 | 100 | 100 | 100 | 100 | 100 | 100 | 80 |
| 149 | POST | 1200 | 50 | 95 | 95 | 95 | 95 | 100 | 95 | 70 |
| | PRE | 1200 | 100 | 100 | 100 | 100 | 100 | 100 | 100 | 100 |
| 150 | POST | 1200 | 0 | 0 | 20 | 0 | 0 | 20 | 0 | 0 |
| | PRE | 1200 | 0 | 0 | 95 | 50 | 10 | 20 | 0 | 0 |
| 151 | POST | 1200 | 40 | 20 | 10 | 20 | 10 | 70 | 75 | 0 |
| | PRE | 1200 | 95 | 100 | 100 | 95 | 100 | 100 | 80 | 20 |
| 152 | POST | 1200 | 50 | 80 | 85 | 80 | 85 | 100 | 80 | 25 |
| | PRE | 1200 | 0 | 100 | 100 | 100 | 100 | 100 | 100 | 0 |
| 153 | POST | 1200 | 90 | 90 | 85 | 90 | 100 | 95 | 95 | 80 |
| | PRE | 1200 | 100 | 100 | 100 | 100 | 100 | 100 | 100 | 100 |
| 154 | POST | 1200 | 70 | 85 | 95 | 90 | 85 | 100 | 85 | 80 |
| | PRE | 1200 | 100 | 100 | 100 | 100 | 100 | 100 | 100 | 80 |
| 155 | POST | 1200 | 25 | 70 | 90 | 25 | 80 | 100 | 70 | 40 |
| | PRE | 1200 | 0 | 100 | 100 | 100 | 100 | 100 | 100 | 0 |

| COMPOUND | TYPE | g/HA | BID | BYG | CRB | FOX | MF | NS | SMT | VEL |
|---|---|---|---|---|---|---|---|---|---|---|
| 156 | POST | 1200 | 0 | 0 | 40 | 0 | 0 | 60 | 0 | 0 |
| | PRE | 1200 | 0 | 25 | 95 | 100 | 100 | 95 | 25 | 20 |
| 157 | POST | 1200 | 10 | 50 | 85 | 20 | 50 | 95 | 75 | 70 |
| | PRE | 1200 | 0 | 100 | 100 | 100 | 100 | 100 | 75 | 10 |
| 158 | POST | 1200 | 40 | 40 | 90 | 70 | 60 | 90 | 80 | 20 |
| | PRE | 1200 | 80 | 70 | 100 | 100 | 95 | 100 | 100 | 0 |
| 159 | POST | 1200 | 75 | 85 | 90 | 85 | 85 | 95 | 85 | 70 |
| | PRE | 1200 | 0 | 100 | 100 | 100 | 100 | 100 | 100 | 60 |
| 160 | POST | 1200 | 10 | 40 | 90 | 10 | 25 | 95 | 20 | 10 |
| | PRE | 1200 | 70 | 75 | 100 | 100 | 95 | 100 | 80 | 25 |
| 161 | POST | 1200 | 10 | 90 | 95 | 90 | 95 | 100 | 90 | 30 |
| | PRE | 1200 | 0 | 100 | 95 | 100 | 100 | 100 | 100 | 20 |
| 162 | POST | 1200 | 10 | 10 | 75 | 10 | 0 | 75 | 10 | 10 |
| | PRE | 1200 | 40 | 0 | 100 | 0 | 20 | 40 | 20 | 0 |
| 163 | POST | 1200 | 40 | 90 | 90 | 70 | 95 | 95 | 75 | 35 |
| | PRE | 1200 | 0 | 100 | 100 | 100 | 100 | 100 | 70 | 0 |
| 164 | POST | 1200 | 20 | 95 | 95 | 40 | 90 | 80 | 70 | 20 |
| | PRE | 1200 | 95 | 100 | 100 | 100 | 100 | 100 | 100 | 25 |
| 165 | POST | 1200 | 20 | 10 | 10 | 10 | 10 | 75 | 10 | 10 |
| | PRE | 1200 | 90 | 25 | 95 | 0 | 0 | 100 | 0 | 0 |
| 166 | POST | 1200 | 20 | 95 | 95 | 100 | 100 | 100 | 90 | 60 |
| | PRE | 1200 | 0 | 100 | 100 | 100 | 100 | 100 | 100 | 100 |
| 167 | POST | 1200 | 0 | 10 | 95 | 10 | 10 | 85 | 25 | 0 |
| | PRE | 1200 | 0 | 40 | 95 | 20 | 10 | 95 | 0 | 0 |

| COMPOUND | TYPE | g/HA | BID | BYG | CRB | FOX | MF | NS | SMT | VEL |
|---|---|---|---|---|---|---|---|---|---|---|
| 168 | POST | 1200 | 85 | 80 | 90 | 80 | 90 | 70 | 70 | 40 |
| | PRE | 1200 | 25 | 95 | 95 | 100 | 100 | 100 | 25 | 25 |
| 169 | POST | 1200 | 0 | 0 | 50 | 0 | 0 | 80 | 0 | 0 |
| | PRE | 1200 | 0 | 90 | 95 | 95 | 10 | 100 | 0 | 0 |
| 170 | POST | 1200 | 0 | 20 | 95 | 0 | 25 | 95 | 50 | 10 |
| | PRE | 1200 | 0 | 90 | 100 | 95 | 90 | 95 | 40 | 0 |
| 171 | POST | 1200 | 10 | 40 | 90 | 40 | 60 | 90 | 70 | 0 |
| | PRE | 1200 | 0 | 90 | 95 | 100 | 95 | 95 | 50 | 0 |
| 172 | POST | 1200 | 25 | 80 | 95 | 90 | 90 | 10 | 95 | 10 |
| | PRE | 1200 | 100 | 100 | 95 | 100 | 100 | 100 | 100 | 10 |
| 173 | POST | 1200 | 25 | 25 | 25 | 20 | 20 | 90 | 20 | 20 |
| | PRE | 1200 | 100 | 100 | 95 | 100 | 100 | 100 | 100 | 40 |
| 174 | POST | 1200 | 0 | 0 | 0 | 0 | 0 | 80 | 10 | 0 |
| | PRE | 1200 | 20 | 70 | 40 | 80 | 70 | 40 | 20 | 10 |
| 175 | POST | 1200 | 0 | 0 | 0 | 0 | 0 | 95 | 10 | 10 |
| | PRE | 1200 | 95 | 95 | 100 | 95 | 100 | 100 | 70 | 20 |
| 176 | POST | 1200 | 90 | 80 | 80 | 70 | 85 | 90 | 90 | 85 |
| | PRE | 1200 | 0 | 100 | 95 | 100 | 95 | 80 | 80 | 80 |
| 177 | POST | 1200 | 0 | 70 | 75 | 0 | 0 | 95 | 60 | 0 |
| | PRE | 1200 | 40 | 100 | 100 | - | 100 | 100 | 90 | 10 |
| 178 | POST | 1200 | 0 | 65 | 75 | 20 | 40 | 95 | 60 | 25 |
| | PRE | 1200 | 20 | 95 | 95 | - | 100 | 100 | 75 | 25 |
| 179 | POST | 1200 | 0 | 20 | 70 | 0 | 20 | 70 | 10 | 0 |
| | PRE | 1200 | 0 | 70 | 100 | - | 100 | 90 | 60 | 20 |

| COMPOUND | TYPE | g/HA | BID | BYG | CRB | FOX | MF | NS | SMT | VEL |
|---|---|---|---|---|---|---|---|---|---|---|
| 180 | POST | 1200 | 80 | 75 | 60 | 65 | 90 | 90 | 75 | 40 |
| | PRE | 1200 | 100 | 100 | 100 | - | 100 | 100 | 100 | 75 |
| 181 | POST | 1200 | 0 | 0 | 0 | 0 | 0 | 10 | 0 | 0 |
| | PRE | 1200 | 100 | 25 | 100 | - | 25 | 70 | 100 | 100 |
| 182 | POST | 1200 | 0 | 80 | 95 | 10 | 0 | 90 | 90 | 10 |
| | PRE | 1200 | 0 | 100 | 100 | - | 100 | 95 | 95 | 95 |
| 183 | POST | 1200 | 40 | 75 | 85 | 70 | 75 | 95 | 80 | 40 |
| | PRE | 1200 | 95 | 95 | 100 | 95 | 95 | 95 | 95 | 60 |
| 184 | POST | 1200 | 20 | 90 | 65 | 75 | 25 | 95 | 85 | 75 |
| | PRE | 1200 | 100 | 95 | 95 | 95 | 95 | 95 | 100 | 40 |

**Claims**

1. A compound of the formula:

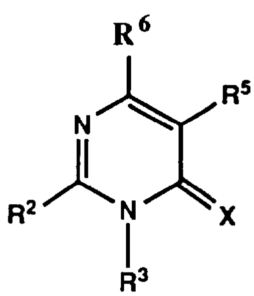

wherein

(a) $R^2$ is a furyl, phenyl, naphthyl, pyridyl, or thienyl group,

each of said groups is optionally substituted with up to three substituents independently selected from a bromo, chloro, fluoro, $(C_1-C_{12})$alkyl, cyclo$(C_3-C_8)$alkyl, $(C_2-C_{12})$alkenyl, cyclo$(C_3-C_8)$alkenyl, $(C_2-C_{12})$alkenyl, halo$(C_1-C_{12})$alkenyl, polyhalo$(C_1-C_{12})$alkyl, halo$(C_2-C_{12})$alkenyl, polyhalo$(C_2-C_{12})$alkenyl, halo$(C_2-C_6)$ alkynyl, polyhalo$(C_2-C_6)$alkynyl, $(C_1-C_{12})$alkoxy, $(C_1-C_{12})$alkylthio, $(C_1-C_{12})$alkylsulfonyl, $(C_1-C_{12})$alkylsulfinul, phenyl, phenyl$(C_1-C_{12})$alkyl, phenyl$(C_2-C_{12})$alkenyl, phenyl$(C_2-C_{12})$alkynyl, cyano, halo$(C_1-C_{12})$alkoxy, $(C_1-C_6)$alkoxy carbonyl 1,3-dioxolan-2-yl, hydroxyimino, or nitro group; and when $R^2$ is pyridyl, such pyridyl group is optionally substituted with oxygen on the nitrogen of the pyridyl group; or R2 is a furyl, phenyl, naphthyl, pyridyl or thienyl group having a fused ring moiety composed of an oxymethyleneoxy or an oxoethyleneoxy link bonded to adjacent carbon atoms of said group;

(b) $R^3$ is a $(C_3-C_4)$alkenyl, $(C_5-C_6)$alkenynyl, $(C_3-C_6)$alkynyl, $(C_1-C_6)$alkoxy$(C_1-C_6)$alkyl, di$(C_1-C_6)$alkoxy$(C_1-C_6)$alkyl, halo$(C_1-C_6)$alkoxy$(C_1-C_6)$alkyl, 2-oxo$(C_2-C_3)$alkyl, trimethylsilyl$(C_3-C_4)$alkynyl or cyano$(C_1-C_6)$alkyl group, each of said $(C_3-C_4)$alkenyl, or $(C_3-C_6)$alkynyl group being optionally substituted with up to five halogens; and

(c) $R^5$ is is a hydrogen, $(C_1-C_5)$alkyl, $(C_3-C_6)$alkenyl, $(C_2-C_6)$alkynyl, halo$(C_1-C_6)$alkyl, polyhalo$(C_1-C_6)$alkyl, halo$(C_2-C_6)$alkenyl, polyhalo$(C_2-C_6)$alkenyl, halo$(C_2-C_6)$alkynyl, polyhalo(C2-C6)alkynyl, halo$(C_1-C_6)$alkoxy, polyhalo$(C_1-C_6)$alkoxy, trimethylsilyl$(C_2-C_3)$alkynyl, $(C_1-C_6)$alkoxy, $(C_1-C_6)$alkylthio, $(C_1-C_3)$alkoxycarbonyl $(C_1-C_3)$alkyl, halo, or cyano group; and

(d) $R^6$ is a hydrogen, halo, $(C_1-C_8)$alkyl, $(C_2-C_6)$alkenyl, $(C_2-C_6)$alkynyl, halo$(C_1-C_6)$alkyl, polyhalo$(C_1-C_6)$ alkyl, halo$(C_2-C_6)$alkenyl, polyhalo$(C_2-C_6)$alkenyl, halo$(C_2-C_6)$alkynyl, polyhalo$(C_2-C_6)$alkynyl, $(C_1-C_6)$alkoxy, $(C_1-C_6)$alkoxo$(C_1-C_4)$alkyl, $(C_1-C_6)$alkylthio, $(C_1-C_3)$alkoxycarbonyl, $(C_1-C_3)$alkoxycarbonyl$(C_1-C_3)$alkyl, $(C_6-C_{10})$aryl, $(C_6-C_{10})$aryl$(C_1-C_4)$alkyl, cyclo$(C_3-C_7)$alkyl, $(C_4-C_5)$heterocyclyl selected from a group consisting of furyl, pyridyl and thienyl, $(C_1-C_3)$alkylamino, di$(C_1-C_3)$alkylamino, di$(C_1-C_3)$alkylaminocarbonyl, halo$(C_1-C_6)$ alkylthio, polyhalo$(C_1-C_6)$alkythio, halo$(C_1-C_6)$alkoxy, polyhalo$(C_1-C_6)$alkoxy, $(C_6-C_{10})$aryloxy or cyano group; the aryl portion of said $(C_6-C_{10})$aryl, $(C_6-C_{10})$ aryl$(C_1-C_4)$alkyl and $(C_6-C_{10})$aryloxy groups being optionally substituted with up to three substituents independently selected from bromo, chloro, fluoro, $(C_1-C_{12})$alkyl, cyco $(C_3-C_8)$alkyl, $(C_2-C_{12})$alkenyl, cyco$(C_3-C_8)$alkenyl, $(C_2-C_{12})$alkynyl, halo$(C_1-C_{12})$alkyl, polyhalo$(C_1-C_{12})$alkyl, halo$(C_2-C_{12})$alkenyl, polyhalo$(C_2-C_{12})$alkenyl, halo$(C_2-C_6)$alkynyl, polyhalo$(C_2-C_6)$alkynyl, $(C_1-C_{12})$alkoxy, $(C_1-C_{12})$alkylthio, $(C_1-C_{12})$alkylsulfonyl, $(C_1-C_{12})$alkylsulfinyl, phenyl, phenyl$(C_1-C_{12})$alkyl, phenyl$(C_2-C_{12})$ alkenyl, phenyl$(C_2-C_{12})$alkynyl, cyano, halo$(C_1-C_{12})$alkoxy, 1,3-dioxalan-2-yl, hydroxyimino, and nitro; and

(e) X is oxygen or sulfur.

**2.** Compound according to claim I wherein $R^2$ is

(a) phenyl, 3-methylphenyl, 3-methoxyphenyl, 3-nitrophenyl, 4-fluorophenyl, 4-chlorophenyl, 3-trifluoromethylphenyl, 3-bromophenyl, 3-chlorophenyl, 3-fluorophenyl, 3-trifluoromethoxyphenyl, 3-cyanophenyl, 3-(1,3-dioxolan-2-yl)-phenyl, 3-(hydroxyimino)phenyl, 2-fluorophenyl, 2-chlorophenyl, 2-trifluoromethoxyphenyl, 3,5-difluorophenyl, 3,5-dichlorophenyl, 2,4-difluorophenyl, 2,5-difluorophenyl, 3-chloro-4-fl uorophenyl, 3,4-difluorophenyl, 3-fluoro3-fluoro-5-trifluoromethylphenyl, or 3,4,53,4,5-trifluorophenyl; or

(b) 6-chloro-2-pyridyl, 3-pyridyl, 1-methyl-3-pyridinium, 5-bromo-3-pyridyl, 5,6-dichloro-3-pyridyl, 5-chloro-3-pyridyl, 1-oxo-3-pyridyl, 4-pyridyl, 2-fluoro-4-pyridyl, 2-methyl-4-pyridyl, 2-methoxy-4-pyridyl, 2-cyano-4-pyridyl, 1-oxo-4-pyridyl, 2-chloro-4-pyridyl, 2-chloro-6-methyl-4-pyridyl, 2,6-difluoro4-pyridyl or 2,6-dichloro-4-pyridyl; or

(c) 2-furyl or 3-furyl; or

(d) 2-thienyl, 3-thienyl, 4-chloro-2-thienyl, 5-chloro-2-thienyl, 5-chloro-3-thienyl, or 2,5-dichloro-3-thienyl.

**3.** Compound according to claim 1 or 2 wherein $R^3$ is allyl, pent-2-ynyl, prop-2-ynyl, but-2-ynyl, methoxymethyl, 2-methoxyethyl, acetonyl, 2,2-dimethoxypropyl, pent-4-en-2-ynyl, 3-chloroallyl, 3-iodopropargyl, 3-trimethylsilyl propargyl, or cyanomethyl.

**4.** Compound according to any preceding claim wherein $R^5$ is hydrogen, fluoro, chloro, methyl, ethyl, n-propyl, i-propyl, prop-2-ynyl, trimethylsilylethynyl, methylthio, methoxy, methoxycarbonylmethyl, allyl, fluoromethyl, or trifluoromethyl.

**5.** Compound according to any preceding claim wherein $R^6$ is

(a) a methyl, ethyl, n-propyl, n-butyl, n-pentyl, n-hexyl, n-heptyl, i-propyl, i-butyl, s-butyl, or t-butyl group; or
(b) a 2-methyl-1-propenyl group; or
(c) a substituted or unsubstituted phenyl group; or
(d) a 3-thienyl, 3-furyl, 2-thienyl or 4-pyridyl group; or
(e) a methoxy or ethoxy group; or
(f) an ethoxycarbonyl group; or
(g) a fluoro, bromo, or chloro group; or
(h) a trifluoromethyl, difluoromethyl, pentafluoroethyl, trichloromethyl, bromomethyl, chloromethyl, or chlorodifluoromethyl group; or
(i) a methylthio group; or
(j) a methoxymethyl group; or
(k) a benzyl group; or
(l) a cyclopropyl, cyclobutyl or cyclopentyl group; or
(m) a dimethylamino group; or
(n) a dimethylaminocarbonyl group; or
(o) hydrogen.

6. Compound according to any preceding claim wherein X is oxygen.

7. Compound according to any one of claims 1 to 5 wherein

X is oxygen;
$R^2$ is phenyl, 3-substituted phenyl or 3,5-disubstituted-phenyl or 3,4,5-trisubstituted-phenyl or 2-substituted-4-pyridyl or 2,6-disubstituted-4-pyridyl or 3-thienyl or 5-substituted-3-thienyl, preferably phenyl, 3-fluorophenyl, 3-chlorophenyl, 3,5-difluorophenyl, 3,4,5-trifluorophenyl, 3,5-dichlorophenyl, 2-chloro-4-pyridyl, 2-fluoro-4-pyridyl, or 2,6-dichloro-4-pyridyl, 3-thienyl or 5-chloro-3-thienyl;
$R^3$ is $(C_3-C_6)$alkynyl, preferably propargyl;
$R^5$ is hydrogen, halo, $(C_1-C_4)$alkyl, polyhalo$(C_1-C_4)$alkyl or $(C_1-C_4)$alkoxy, preferably hydrogen, methyl, ethyl, methoxy, fluoro or chloro; and
$R^6$ is hydrogen, halo, $(C_1-C_4)$alkyl, phenyl optionally substituted as defined for $R^6$ in claim 1, polyhalo$(C_1-C_4)$alkyl or $(C_1-C_4)$alkoxy; preferably hydrogen, methyl, ethyl, isopropyl, n-propyl, n-butyl, s-butyl, i-butyl, t-butyl, difluoromethyl, trifluoromethyl, phenyl, chloro, bromo, or fluoro.

8. Compound according to claim 1 wherein $R^2$ is 2-furyl, phenyl, 3-fluorophenyl, 3-chlorophenyl, 3,5-difluorophenyl, 3,5-dichlorophenyl, 2-chloro-4-pyridyl, 2-fluoro-4-pyridyl or 2,6-dichloro-4-pyridyl.

9. Compound according to any preceding claim which is

5,6-diethyl-2-phenyl-3-propargyl-4(3H)-pyrimidinone;
5-ethyl-2-phenyl-3-propargyl-6-trifluoromethyl-4(3H)-pyrimidinone;
5-ethyl-6-isopropyl-2-phenyl-3-propargyl-4(3H)-pyrimidinone;
6-chloro-5-ethyl-2-phenyl-3-propargyl-4(3H)-pyrimidinone;
5,6-diethyl-2-(3-fluorophenyl)-3-propargyl-4(3H)-pyrimidinone;
2-(2,6-dichloro-4-pyridyl)-5,6-diethyl-3-propargyl-4(3H)-pyrimidinone;
5,6-diethyl-2-(3,5-difluorophenyl)-3-propargyl-4(3H)-pyrimidinone;
5-ethyl-2-phenyl-3-propargyl-6-(n-propyl)-4(3H)-pyrimidinone;
6-difluoromethyl-5-ethyl-2-phenyl-3-propargyl-4(3H)-pyrimidinone;
6-ethyl-5-methoxy-2-phenyl-3-propargyl-4(3H)-pyrimidinone.

10. A herbicidal composition comprising a herbicidally effective amount of a compound as defined in any preceding claim, and an agronomically acceptable carrier.

11. A method of controlling a weed comprising applying a herbicidally effective amount of a composition or compound as defined in any preceding claim to said weed or to the locus of said weed or to the growth medium of said weed.

12. The use of a compound or composition as defined in any of claims 1 to 10 as a herbicide.

**Patentansprüche**

1. Verbindung der Formel:

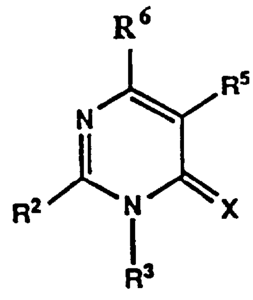

wobei

(a) $R^2$ eine Furyl-, Phenyl, Naphthyl-, Pyridyl- oder Thienyl-Gruppe ist, wobei jede dieser Gruppen gegebenenfalls substituiert ist mit bis zu drei Substituenten, unabhängig ausgewählt aus einer Brom-, Chlor-, Fluor-, ($C_1$-$C_{12}$)alkyl-, Cyclo($C_3$-$C_8$) alkyl-, ($C_2$-$C_{12}$) alkenyl-, cyclo($C_3$-$C_8$)alkenyl-, ($C_2$-$C_{12}$)alkynyl-, Halogen($C_1$-$C_{12}$) alkyl-, Polyhalogen($C_1$-$C_{12}$)alkyl-, Halogen ($C_2$-$C_{12}$) alkenyl, Polyhalogen($C_2$-$C_{12}$)alkenyl, Halogen($C_2$-$C_6$)-alkynyl-, Polyhalogen($C_2$-$C_6$) alkynyl-, ($C_1$-$C_{12}$)-alkoxy-, ($C_1$-$C_{12}$)alkylthio-, ($C_1$-$C_{12}$)alkylsulfonyl-, ($C_1$-$C_{12}$)alkylsulfinyl-, Phenyl-, Phenyl-($C_1$-$C_{12}$)alkyl-, Phenyl($C_2$-$C_{12}$)alkenyl-, Phenyl-($C_2$-$C_{12}$)alkynyl-, Cyano-, Halogen $C_1$-$C_{12}$)alkoxy-, ($C_1$-$C_6$)alkoxycarbonyl-, 1,3-Dioxolan-2-yl-, Hydroxyimino- oder Nitrogruppe; und wenn $R^2$ Pyridyl ist, ist solche eine Pyridylgruppe gegebenenfalls substituiert mit Sauerstoff am Stickstoff der Pyridylgruppe; oder $R^2$ ist eine Furyl-, Phenyl- Naphthyl-, Pyridyl- oder Thienyl-Gruppe mit einer kondensierten Ringkomponente, zusammengesetzt aus einer Oxymethylenoxy- oder einer Oxoethylenoxy-Bindung, gebunden an benachbarte Kohlenstoffatome dieser Gruppe;
(b) $R^3$ eine ($C_3$-$C_4$)alkenyl-, ($C_5$-$C_6$)alkenynyl-, ($C_3$-$C_6$)alkynyl-, ($C_1$-$C_6$)alkoxy($C_1$-$C_6$)alkyl-, Di($C_1$-$C_6$)alkoxy ($C_1$-$C_6$)alkyl, Halogen($C_1$-$C_6$)alkoxy($C_1$-$C_6$)alkyl-, 2-Oxo($C_2$-$C_3$)alkyl-, Trimethylsilyl-($C_3$-$C_4$)alkynyl- oder Cyano($C_1$-$C_6$)alkyl-Gruppe ist, wobei diese ($C_3$-$C_4$)alkenyl- oder ($C_3$-$C_6$)-alkynyl-Gruppe gegebenenfalls mit bis zu 5 Halogenen substituiert ist; und
(c) $R^5$ eine Wasserstoff-, ($C_1$-$C_5$)alkyl-, ($C_3$-$C_6$)alkenyl-, ($C_2$-$C_6$)alkynyl-, Halogen($C_1$-$C_6$)-alkyl-, Polyhalogen ($C_1$-$C_6$)alkyl-, Halogen($C_2$-$C_6$)alkenyl-, Polyhalogen($C_2$-$C_6$)alkenyl-, Halogen($C_2$-$C_6$)alkynyl-, Polyhalogen ($C_2$-$C_6$)alkynyl-, Halogen($C_1$-$C_6$)alkoxy-, Polyhalogen($C_1$-$C_6$)alkoxy-, Trimethylsilyl($C_2$-$C_3$)alkynyl-, ($C_1$-$C_6$) alkoxy-, ($C_1$-$C_6$)alkylthio-, ($C_1$-$C_3$)alkoxycarbonyl($C_1$-$C_3$)alkyl-, Halogen- oder Cyano-Gruppe ist; und
(d) $R^6$ eine Wasserstoff-, Halogen($C_1$-$C_8$)alkyl-, ($C_2$-$C_6$)alkenyl, ($C_2$-$C_6$)alkynyl-, Halogen($C_1$-$C_6$)-alkyl-, Polyhalogen($C_1$-$C_6$)alkyl-, Halogen($C_2$-$C_6$)alkenyl-, Polyhalogen($C_2$-$C_6$)alkenyl-, Halogen($C_2$-$C_6$)alkynyl-, Polyhalogen($C_2$-$C_6$)alkynyl-, ($C_1$-$C_6$)alkoxy-, ($C_1$-$C_6$)alkoxy-($C_1$-$C_4$)alkyl-, ($C_1$-$C_6$)alkylthio-, ($C_1$-$C_3$)alkoxycarbonyl-, ($C_1$-$C_3$)alkoxycarbonyl($C_1$-$C_3$)alkyl-, ($C_6$-$C_{10}$)aryl-, ($C_6$-$C_{10}$)aryl($C_1$-$C_4$)alkyl-, Cyclo($C_3$-$C_7$)alkyl-, ($C_4$-$C_5$)heterocyclyl-, ausgewählt aus einer Gruppe, bestehend aus Furyl, Pyridyl und Thienyl, ($C_1$-$C_3$)alkylamino-, Di ($C_1$-$C_3$)alkylamino-, Di($C_1$-$C_3$)alkylaminocarbonyl-, Halogen($C_1$-$C_6$)alkyl-thio-, Polyhalogen($C_1$-$C_6$)alkylthio-, Halogen($C_1$-$C_6$)alkoxy-, Polyhalogen($C_1$-$C_6$)alkoxy-, ($C_6$-$C_{10}$)-aryloxy- oder Cyangruppe ist; wobei der Arylanteil der ($C_6$-$C_{10}$)aryl-, ($C_6$-$C_{10}$)aryl($C_1$-$C_4$)-alkyl- und ($C_6$-$C_{10}$)aryloxy-Gruppen gegebenenfalls substituiert ist mit bis zu drei Substituenten, unabhängig ausgewählt aus Brom, Chlor, Fluor, ($C_1$-$C_{12}$)alkyl, Cyclo($C_3$-$C_8$) alkyl, ($C_2$-$C_{12}$)alkenyl, Cyclo($C_3$-$C_8$)alkenyl, ($C_2$-$C_{12}$)-alkynyl, Halogen($C_1$-$C_{12}$)alkyl, Polyhalogen ($C_1$-$C_{12}$)alkyl, Halogen($C_2$-$C_{12}$)alkenyl, Polyhalogen($C_2$-$C_{12}$)alkenyl, Halogen($C_2$-$C_6$)alkynyl, Polyhalogen($C_2$-$C_6$)alkynyl, ($C_1$-$C_{12}$)alkoxy, ($C_1$-$C_{12}$)-alkylthio, ($C_1$-$C_{12}$)alkylsulfonyl, ($C_1$-$C_{12}$)-alkylsulfinyl, Phenyl, Phenyl($C_1$-$C_{12}$)alkyl, Phenyl($C_2$-$C_{12}$)alkenyl, Phenyl($C_2$-$C_{12}$)alkynyl, Cyan, Halogen($C_1$-$C_{12}$)alkoxy, 1,3-Dioxalan-2-yl, Hydroxyimino und Nitro; und
(e) X Sauerstoff oder Schwefel ist.

**2.** Verbindung nach Anspruch 1, wobei $R^2$

(a) Phenyl, 3-Methylphenyl, 3-Methoxyphenyl, 3-Nitrophenyl, 4-Fluorphenyl, 4-Chlorphenyl, 3-Trifluormethylphenyl, 3-Bromphenyl, 3-Chlorphenyl, 3-Fluorphenyl, 3-Trifluormethoxyphenyl, 3-Cyanphenyl, 3-(1,3-Dioxolan-2-yl)phenyl, 3-(Hydroxyimino)phenyl, 2-Fluorphenyl, 2-Chlorphenyl, 2-Trifluormethoxyphenyl, 3,5-Difluorphenyl, 3,5-Dichlorphenyl, 2,4-Difluorphenyl, 2,5-Difluorphenyl, 3-Chlor-4-fluorphenyl, 3,4-Difluorphenyl, 3-Fluor-5-trifluormethylphenyl oder 3,4,5-Trifluorphenyl; oder
(b) 6-Chlor-2-pyridyl, 3-Pyridyl, 1-Methyl-3-pyridinium, 5-Brom-3-pyridyl, 5,6-Dichlor-3-pyridyl, 5-Chlor-3-pyridyl, 1-Oxo-3-pyridyl, 4-Pyridyl, 2-Fluor-4-pyridyl, 2-Methyl-4-pyridyl, 2-Methoxy-4-pyridyl, 2-Cyan-4-pyridyl, 1-Oxo-4-pyridyl, 2-Chlor-4-pyridyl, 2-Chlor-6-methyl-4-pyridyl, 2,6-Difluor-4-pyridyl oder 2,6-Dichlor-4-pyridyl; oder
(c) 2-Furyl oder 3-Furyl; oder
(d) 2-Thienyl, 3-Thienyl, 4-chlor-2-thienyl, 5-Chlor-2-thienyl, 5-chlor-3-thienyl oder 2,5-Dichlor-3-thienyl ist.

**3.** Verbindung nach Anspruch 1 oder 2, wobei $R^3$ Allyl, Pent-2-ynyl, Prop-2-ynyl, But-2-ynyl, Methoxymethyl, 2-Methoxyethyl, Acetonyl, 2,2-Dimethoxypropyl, Pent-4-en-2-ynyl, 3-Chlorallyl, 3-Iodproparqyl, 3-Trimethylsilyl, Propargyl oder Cyanmethyl ist.

**4.** Verbindung nach einem der vorhergehenden Ansprüche, wobei $R^5$ Wasserstoff, Fluor, Chlor, Methyl, Ethyl, n-Propyl, i-Propyl, Prop-2-ynyl, Trimethylsilylethynyl, Methylthio, Methoxy, Methoxycarbonylmethyl, Allyl, Fluormethyl oder Trifluormethyl.

5. Verbindung nach einem der vorhergehenden Ansprüche, wobei $R^6$

(a) eine Methyl-, Ethyl-, n-Propyl-, n-Butyl-, n-Pentyl-, n-Hexyl-, n-Heptyl-, i-Propyl-, 1-Butyl-, s-Butyl- oder t-Butyl-Gruppe ist; oder
(b) eine 2-Methyl-l-propenyl-Gruppe; oder
(c) eine substituierte oder unsubstituierte Phenylgruppe; oder
(d) eine 3-Thienyl-, 3-Furyl-, 2-Thienyl- oder 4-Pyridyl-Gruppe; oder
(e) eine Methoxy- oder Ethoxy-Gruppe; oder
(f) eine Ethoxycarbonyl-Gruppe; oder
(g) eine Fluor-, Brom- oder Chlor-Gruppe; oder
(h) eine Trifluormethyl-, Difluormethyl-, Pentafluorethyl-, Trichlormethyl-, Brommethyl-, Chlormethyl- oder Chlordifluormethyl-Gruppe; oder
(i) eine Methylthio-Gruppe; oder
(j) eine Methoxymethyl-Gruppe; oder
(k) eine Benzyl-Gruppe; oder
(1) eine Cyclopropyl-, Cyclobutyl- oder Cyclopentyl-Gruppe; oder
(m) eine Dimethylamino-Gruppe; oder
(n) eine Dimethylaminocarbonyl-Gruppe; oder
(o) Wasserstoff ist.

6. Verbindung gemäß einem der vorhergehenden Ansprüche, wobei X Sauerstoff ist.

7. Verbindung gemäß einem der Ansprüche 1 bis 5, wobei X Sauerstoff ist;

$R^2$ Phenyl, 3-substituiertes Phenyl oder 3,5-disubstituiertes Phenyl oder 3,4,5-trisubstituiertes Phenyl oder 2-substituiertes-4-Pyridyl oder 2,6-disubstituiertes-4-Pyridyl oder 3-Thienyl oder 5-substituiertes-3-Thienyl, vorzugsweise Phenyl, 3-Fluorphenyl, 3-Chlorphenyl, 3,5-Difluorphenyl, 3,4,5-Trifluorphenyl, 3,5-Dichlorphenyl, 2-Chlor-4-pyridyl, 2-Fluor-4-pyridyl oder 2,6-Dichlor-4-pyridyl, 3-Thienyl oder 5-chlor-3-thienyl ist;
$R^3$ $(C_3\text{-}C_6)$alkynyl, vorzugsweise Propargyl ist;
$R^5$ Wasserstoff, Halogen, $(C_1\text{-}C_4)$alkyl, Polyhalogen$(C_1\text{-}C_4)$alkyl oder $(C_1\text{-}C_4)$alkoxy, vorzugsweise Wasserstoff, Methyl, Ethyl, Methoxy, Fluor oder Chlor ist; und
$R^6$ Wasserstoff, Halogen, $(C_1\text{-}C_4)$alkyl, Phenyl, gegebenenfalls substituiert wie es für $R^6$ in Anspruch 1 definiert ist, Polyhalogen$(C_1\text{-}C_4)$alkyl oder $(C_1\text{-}C_4)$alkoxy; vorzugsweise Wasserstoff, Methyl, Ethyl, Isopropyl, n-Propyl, n-Butyl, s-Butyl, i-Butyl, t-Butyl, Difluormethyl, Trifluormethyl, Phenyl, Chlor, Brom oder Fluor ist.

8. Verbindung nach Anspruch 1, wobei $R^2$ 2-Furyl, Phenyl, 3-Fluorphenyl, 3-Chlorphenyl, 3,5-Difluorphenyl, 3,5-Dichlorphenyl, 2-chlor-4-pyridyl, 2-Fluor-4-pyridyl oder 2,6-Dichlor-4-pyridyl ist.

9. Verbindung nach einem der vorhergehenden Ansprüche, welche

5,6-Diethyl-2-phenyl-3-propargyl-4(3H)-pyrimidinon; 5-Ethyl-2-phenyl-3-propargyl-6-trifluormethyl-4(3H)-pyrimidinon;
5-Ethyl-6-isopropyl-2-phenyl-3-propargyl-4(3H)-pyrimidinon;
6-chlor-5-ethyl-2-phenyl-3-propargyl-4(3H)-pyrimidinon;
5,6-Diethyl-2-(3-fluorphenyl)-3-propargyl-4(3H)-pyrimidinon;
2-(2,6-Dichlor-4-pyridyl)-5,6-diethyl-3-propargyl-4(3H)-pyrimidinon;
5,6-Diethyl-2-(3,5-difluorphenyl)-3-propargyl-4(3H)-pyrimidinon;
5-Ethyl-2-phenyl-3-propargyl-6-(n-propyl)-4(3H)-pyrimidinon;
6-Difluormethyl-5-ethyl-2-phenyl-3-propargyl-4(3H)-pyrimidinon;
6-Ethyl-5-methoxy-2-phenyl-3-propargyl-4(3H)-pyrimidinon ist.

10. Herbizidale Zusammensetzung, umfassend eine herbizidal wirksame Menge einer Verbindung, wie sie in einem der vorhergehenden Ansprüche definiert ist, und einen für die Landwirtschaft verträglichen Träger.

11. Verfahren zur Unkrautbekämpfung, umfassend Aufbringen einer herbizidal wirksamen Menge einer Zusammensetzung oder Verbindung, wie in einem der vorhergehenden Ansprüche definiert, auf dieses Unkraut oder auf eine Stelle dieses Unkrauts oder in ein Wachstumsmedium dieses Unkrauts.

**12.** Verwendung einer Verbindung oder Zusammensetzung, wie in einem der Ansprüche 1 bis 10 definiert, als Herbizid.

**Revendications**

**1.** Composé de formule:

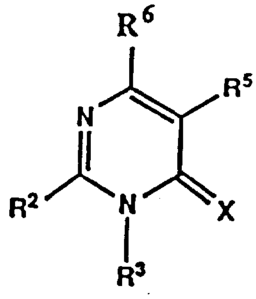

dans laquelle

(a) $R^2$ est un groupe furyle, phényle, naphtyle, pyridyle ou thiényle, chacun desdits groupes étant éventuellement substitué par au plus trois substituants choisis indépendamment parmi les groupes bromo, chloro, fluoro, alkyle en $C_1$-$C_{12}$, cycloalkyle en $C_3$-$C_8$, alcényle en $C_2$-$C_{12}$, cycloalcényle en $C_3$-$C_8$, alcynyle en $C_2$-$C_{12}$, halogénoalkyle en $C_1$-$C_{12}$, polyhalobénoalkyle en $C_1$-$C_{12}$, halogénoalcényle en $C_2$-$C_{12}$, polyhalogénoalcényle en $C_2$-$C_{12}$, halogénoalcynyle en $C_2$-$C_6$, polyhalogénoalcynyle en $C_2$-$C_6$, alcoxy en $C_1$-$C_{12}$, alkylthio en $C_1$-$C_{12}$, alkylsulfonyle en $C_1$-$C_{12}$, alkylsulfinyle en $C_1$-$C_{12}$, phényle, phényl(alkyle en $C_1$-$C_{12}$), phényl(alcényle en $C_2$-$C_{12}$), phényl(alcynyle en $C_2$-$C_{12}$), cyano, halogénoalcoxy en $C_1$-$C_{12}$, alcoxy(en $C_1$-$C_6$)carbonyle, 1,3-dioxolane-2-yle, hydroxyimino ou nitro; et, lorsque $R^2$ est un groupe pyridyle, ce groupe pyridyle est éventuellement substitué par un atome d'oxygène sur l'atome d'azote du groupe pyridyle; ou bien $R^2$ est un groupe furyle, phényle, naphtyle, pyridyle ou thiényle comportant un groupement à noyaux condensés composé d'une liaison oxyméthylèneoxy ou oxoéthylèneoxy liée à des atomes de carbone adjacents dudit groupe;

(b) $R^3$ est un groupe alcényle en $C_3$-$C_4$, alcénynyle en $C_5$-$C_6$, alcynyle en $C_3$-$C_6$, alcoxy(en $C_1$-$C_6$) (alkyle en $C_1$-$C_6$), di(alcoxy en $C_1$-$C_6$) (alkyle en $C_1$-$C_6$), halogénoalcoxy(en $C_1$-$C_6$) (alkyle en $C_1$-$C_6$), 2-oxoalkyle en $C_2$-$C_3$, triméthylsilyl(alcynyle en $C_3$-$C_4$) ou cyano(alkyle en $C_1$-$C_6$), chacun desdits groupes alcényle en $C_3$-$C_4$ ou alcynyle en $C_3$-$C_6$ étant éventuellement substitué par au plus cinq atomes d'halogène; et

(c) $R^5$ est un atome d'hydrogène, ou un groupe alkyle en $C_1$-$C_5$, alcényle en $C_3$-$C_6$, alcynyle en $C_2$-$C_6$, halogénoalkyle en $C_1$-$C_6$, polyhalogénoalkyle en $C_1$-$C_6$, halogénoalcényle en $C_2$-$C_6$, polyhalogénoalcényle en $C_2$-$C_6$, halogénoalcynyle en $C_2$-$C_6$, polyhalogénoalcynyle en $C_2$-$C_6$, halogénoalcoxy en $C_1$-$C_6$, polyhalogénoalcoxy en $C_1$-$C_6$, triméthylsilyl(alcynyle en $C_2$-$C_3$), alcoxy en $C_1$-$C_6$, alkylthio en $C_1$-$C_6$, alcoxy(en $C_1$-$C_3$)carbonyl (alkyle en $C_1$-$C_3$), halogéno ou cyano; et

(d) $R^6$ est un atome d'hydrogène ou un groupe halogéno, alkyle en $C_1$-$C_8$, alcényle en $C_2$-$C_6$, alcynyle en $C_2$-$C_6$, halogénoalkyle en $C_1$-$C_6$, polyhalogénoalkyle en $C_1$-$C_6$, halogénoalcényle en $C_2$-$C_6$, polyhalogénoalcényle en $C_2$-$C_6$, halogénoalcynyle en $C_2$-$C_6$, polyhalogénoalcynyle en $C_2$-$C_6$, alcoxy en $C_1$-$C_6$, alcoxy(en $C_1$-$C_6$)(alkyle en $C_1$-$C_4$), alkylthio en $C_1$-$C_6$, alcoxy(en $C_1$-$C_3$)carbonyle, alcoxy(en $C_1$-$C_3$)carbonyl (alkyle en $C_1$-$C_3$), aryle en $C_6$-$C_{10}$, aryl(en $C_6$-$C_{10}$) (alkyle en $C_1$-$C_4$), cycloalkyle en $C_3$-$C_7$, hétérocyclyle en $C_4$-$C_5$ choisi dans un ensemble constitué par les groupes furyle, pyridyle et thiényle, alkylamino en $C_1$-$C_3$, di(alkylamino en $C_1$-$C_3$), di(alkyl en $C_1$-$C_3$)aminocarbonyle, halogénoalkylthio en $C_1$-$C_6$, polyhalogénoalkylthio en $C_1$-$C_6$, halogénoalcoxy en $C_1$-$C_6$, polyhalogénoalcoxy en $C_1$-$C_6$, aryloxy en $C_6$-$C_{10}$ ou cyano; la partie aryle desdits groupes aryle en $C_6$-$C_{10}$, aryl(en $C_6$-$C_{10}$)(alkyle en $C_1$-$C_4$) et aryloxy en $C_6$-$C_{10}$ étant éventuellement substituée par au plus trois substituants choisis indépendamment parmi les groupes bromo, chloro, fluoro, alkyle en $C_1$-$C_{12}$, cycloalkyle en $C_3$-$C_8$, alcényle en $C_2$-$C_{12}$, cycloalcényle en $C_3$-$C_8$, alcynyle en $C_2$-$C_{12}$, halogénoalkyle en $C_1$-$C_{12}$, polyhalogénoalkyle en $C_1$-$C_{12}$, halogénoalcényle en $C_2$-$C_{12}$, polyhalogénoalcényle en $C_2$-$C_{12}$, halogénoalcynyle en $C_2$-$C_6$, polyhalogénoalcynyle en $C_2$-$C_6$, alcoxy en $C_1$-$C_{12}$, alkylthio en $C_1$-$C_{12}$, alkylsulfonyle en $C_1$-$C_{12}$, alkylsulfinyle en $C_1$-$C_{12}$, phényle, phényl(alkyle en $C_1$-$C_{12}$), phényl(alcényle en $C_2$-$C_{12}$), phényl(alcynyle en $C_2$-$C_{12}$), cyano, halogénoalcoxy en $C_1$-$C_{12}$, 1,3-dioxolane-2-yle, hydroxyimino ou nitro; et

(e) X est un atome d'oxygène ou de soufre.

**2.** Composé selon la revendication 1, dans lequel $R^2$ est

(a) un groupe phényle, 3-méthylphényle, 3-méthoxyphényle, 3-nitrophényle, 4-fluorophényle, 4-chlorophényle, 3-trifluorométhylphényle, 3-bromophényle, 3-chlorophényle, 3-fluorophényle, 3-trifluorométhoxyphényle, 3-cyanophényle, 3-(1,3-dioxolane-2-yl)phényle, 3-(hydroxyimino)phényle, 2-fluorophényle, 2-chlorophényle, 2-trifluorométhoxyphényle, 3,5-difluorophényle, 3,5-dichlorophényle, 2,4-difluorophényle, 2,5-difluorophényle, 3-chloro-4-fluorophényle, 3,4-difluorophényle, 3-fluoro-5-trifluorométhylphényle ou 3,4,5-trifluorophényle; ou
(b) un groupe 6-chloro-2-pyridyle, 3-pyridyle, 1-méthyl-3-pyridinium, 5-bromo-3-pyridyle, 5,6-dichloro-3-pyridyle, 5-chloro-3-pyridyle, 1-oxo-3-pyridyle, 4-pyridyle, 2-fluoro-4-pyridyle, 2-méthyl-4-pyridyle, 2-méthoxy-4-pyridyle, 2-cyano-4-pyridyle, 1-oxo-4-pyridyle, 2-chloro-4-pyridyle, 2-chloro-6-méthyl-4-pyridyle, 2,6-difluoro-4-pyridyle ou 2,6-dichloro-4-pyridyle; ou
(c) un groupe 2-furyle ou 3-furyle; ou
(d) un groupe 2-thiényle, 3-thiényle, 4-chloro-2-thiényle, 5-chloro-2-thiényle, 5-chloro-3-thiényle ou 2,5-dichloro-3-thiényle.

**3.** Composé selon la revendication 1 ou 2, dans lequel $R^3$ est un groupe allyle, pent-2-ynyle, prop-2-ynyle, but-2-ynyle, méthoxyméthyle, 2-méthoxyéthyle, acétonyle, 2,2-diméthoxypropyle, pent-4-ène-2-ynyle, 3-chloroallyle, 3-iodopropargyle, 3-triméthylsilylpropargyle ou cyanométhyle.

**4.** Composé selon l'une quelconque des revendications précédentes, dans lequel $R^5$ est un atome d'hydrogène ou un groupe fluoro, chloro, méthyle, éthyle, n-propyle, isopropyle, prop-2-ynyle, triméthylsilyléthynyle, méthylthio, méthoxy, méthoxycarbonylméthyle, allyle, fluorométhyle ou trifluorométhyle.

**5.** Composé selon l'une quelconque des revendications précédentes, dans lequel $R^6$ est

(a) un groupe méthyle, éthyle, n-propyle, n-butyle, n-pentyle, n-hexyle, n-heptyle, isopropyle, isobutyle, s-butyle ou t-butyle; ou
(b) un groupe 2-méthyl-1-propényle; ou
(c) un groupe phényle substitué ou non substitué; ou
(d) un groupe 3-thiényle, 3-furyle, 2-thiényle ou 4-pyridyle; ou
(e) un groupe méthoxy ou éthoxy; ou
(f) un groupe éthoxycarbonyle; ou
(g) un groupe fluoro, bromo ou chloro; ou
(h) un groupe trifluorométhyle, difluorométhyle, pentafluoroéthyle, trichlorométhyle, bromométhyle, chlorométhyle ou chlorodifluorométhyle; ou
(i) un groupe méthylthio; ou
(j) un groupe méthoxyméthyle; ou
(k) un groupe benzyle; ou
(1) un groupe cyclopropyle, cyclobutyle ou cyclopentyle; ou
(m) un groupe diméthylamino; ou
(n) un groupe diméthylaminocarbonyle; ou
(o) un atome d'hydrogène.

**6.** Composé selon l'une quelconque des revendications précédentes, dans lequel X est un atome d'oxygène.

**7.** Composé selon l'une quelconque des revendications 1 à 5, dans lequel

X est un atome d'oxygène;
$R^2$ est un groupe phényle, phényle 3-substitué ou phényle 3,5-disubstitué ou phényle 3,4,5-trisubstitué ou 4-pyridyle 2-substitué ou 4-pyridyle 2,6-disubstitué ou 3-thiényle ou 3-thiényle 5-substitué, de préférence phényle, 3-fluorophényle, 3-chlorophényle, 3,5-difluorophényle, 3,4,5-trifluorophényle, 3,5-dichlorophényle, 2-chloro-4-pyridyle, 2-fluoro-4-pyridyle ou 2,6-dichloro-4-pyridyle, 3-thiényle ou 5-chloro-3-thiényle;
$R^3$ est un groupe alcynyle en $C_3$-$C_6$, de préférence propargyle;
$R^5$ est un atome d'hydrogène ou un groupe halogéno, alkyle en $C_1$-$C_4$, polyhalogénoalkyle en $C_1$-$C_4$ ou alcoxy en $C_1$-$C_4$, de préférence un atome d'hydrogène ou un groupe méthyle, éthyle, méthoxy, fluoro ou chloro; et
$R^6$ est un atome d'hydrogène ou un groupe halogéno, alkyle en $C_1$-$C_4$, phényle éventuellement substitué de la manière définie pour $R^6$ dans la revendication 1, polyhalogénoalkyle en $C_1$-$C_4$ ou alcoxy en $C_1$-$C_4$; de

préférence un atome d'hydrogène ou un groupe méthyle, éthyle, isopropyle, n-propyle, n-butyle, s-butyle, isobutyle, t-butyle, difluorométhyle, trifluorométhyle, phényle, chloro, bromo ou fluoro.

**8.** Composé selon la revendication 1, dans lequel $R^2$ est un groupe 2-furyle, phényle, 3-fluorophényle, 3-chlorophényle, 3,5-difluorophényle, 3,5-dichlorophényle, 2-chloro-4-pyridyle, 2-fluoro-4-pyridyle ou 2,6-dichloro-4-pyridyle.

**9.** Composé selon l'une quelconque des revendications précédentes, qui est:

la 5,6-diéthyl-2-phényl-3-propargyl-4(3H)-pyrimidinone; la 5-éthyl-2-phényl-3-propargyl-6-trifluorométhyl-4(3H)-pyrimidinone;
la 5-éthyl-6-isopropyl-2-phényl-3-propargyl-4(3H)-pyrimidinone;
la 6-chloro-5-éthyl-2-phényl-3-propargyl-4(3H)-pyrimidinone;
la 5,6-diéthyl-2-(3-fluorophényl)-3-propargyl-4(3H)-pyrimidinone;
la 2-(2,6-dichloro-4-pyridyl)-5,6-diéthyl-3-propargyl-4(3H)-pyrimidinone;
la 5,6-diéthyl-2-(3,5-difluorophényl)-3-propargyl-4(3H)-pyrimidinone;
la 5-éthyl-2-phényl-3-propargyl-6-(n-propyl)-4(3H)-pyrimidinone;
la 6-difluorométhyl-5-éthyl-2-phényl-3-propargyl-4(3H)-pyrimidinone;
la 6-éthyl-5-méthoxy-2-phényl-3-propargyl-4(3H)-pyrimidinone.

**10.** Composition herbicide comprenant une quantité efficace en tant qu'herbicide d'un composé tel que défini dans l'une quelconque des revendications précédentes, et un véhicule acceptable en agronomie.

**11.** Procédé pour lutter contre une mauvaise herbe, comprenant l'application d'une quantité efficace en tant qu'herbicide d'une composition ou d'un composé, tel que défini dans l'une quelconque des revendications précédentes, sur ladite mauvaise herbe ou sur le lieu de ladite mauvaise herbe ou sur le milieu de croissance de ladite mauvaise herbe.

**12.** Utilisation comme herbicide d'un composé ou d'une composition, tel que défini dans l'une quelconque des revendications 1 à 10.